# EUROPEAN PATENT SPECIFICATION

(11) **EP 3 102 609 B1**
(45) Date of publication and mention of the grant of the patent: **28.08.2024**
(21) Application number: 15746399.3
(22) Date of filing: 04.02.2015
(51) Int. Cl.: C12N 5/0783, A61K 39/00, A61K 38/17, C07K 14/725, A61P 35/00

(54) **METHODS FOR PRODUCING AUTOLOGOUS T CELLS USEFUL TO TREAT B CELL MALIGNANCIES AND OTHER CANCERS AND COMPOSITIONS THEREOF**
VERFAHREN ZUR HERSTELLUNG AUTOLOGER T-ZELLEN ZUR BEHANDLUNG VON B-ZELL-MALIGNOMEN UND ANDEREN KREBSARTEN UND ZUSAMMENSETZUNGEN DAVON
MÉTHODES DE PRODUCTION DE LYMPHOCYTES T AUTOLOGUES UTILISÉS POUR TRAITER LES TUMEURS MALIGNES À LYMPHOCYTES B ET D'AUTRES CANCERS, ET COMPOSITIONS ASSOCIÉES

(30) Priority: 04.02.2014 US 201461935833 P
(43) Date of publication of application: 14.12.2016
(60) Divisional application: 17154769.8
(73) Proprietor: The United States of America, as Represented by The Secretary, Department of Health and Human Services, Bethesda, Maryland 20892-7788 (US); Kite Pharma, Inc., Santa Monica, CA 90404 (US)
(72) Inventor: BETTER, Marc, Santa Monica, California 90404 (US); FELDMAN, Steven A., Bethesda, Maryland 20892-1201 (US); ROSENBERG, Steven A., Bethesda, Maryland 20892-1201 (US)
(74) Representative: Pohlman, Sandra M.
(86) International application number: PCT/US2015/014520
(87) International publication number: WO 2015/120096

(56) References cited:
- EP-A1- 2 267 118
- WO-A1-2012/138475
- WO-A1-2013/072288
- WO-A1-2015/162211
- WO-A2-2013/040557
- LAMERS C H J ET AL: "Protocol for gene transduction and expansion of human T lympocytes for clinical immunogene therapy of cancer", CANCER GENE THERAPY, APPLETON & LANGE, GB, vol. 9, 1 January 2002 (2002-01-01), pages 613 - 623, XP003020145, ISSN: 0929-1903, DOI: 10.1038/SJ.CGT.7700477
- C.H.J. LAMERS ET AL: "Retronectin -assisted retroviral transduction of primary human T lymphocytes under good manufacturing practice conditions: tissue culture bag critically determines cell yield", CYTOTHERAPY, vol. 10, no. 4, 1 January 2008 (2008-01-01), GB, pages 406 - 416, XP055390268, ISSN: 1465-3249, DOI: 10.1080/14653240801982961
- BARBARA TUMAINI ET AL: "Simplified process for the production of anti-CD19-CAR-engineered T cells", CYTOTHERAPY, vol. 15, no. 11, 1 November 2013 (2013-11-01), pages 1406 - 1415, XP055211897, ISSN: 1465-3249, DOI: 10.1016/j.jcyt.2013.06.003
- A D KAISER ET AL: "Towards a commercial process for the manufacture of genetically modified T cells for therapy", CANCER GENE THERAPY, vol. 22, no. 2, 23 January 2015 (2015-01-23), pages 72 - 78, XP055211554, ISSN: 0929-1903, DOI: 10.1038/cgt.2014.78
- TANGYING LILY LU ET AL: "A Rapid Cell Expansion Process for Production of Engineered Autologous CAR-T Cell Therapies", HUMAN GENE THERAPY METHODS, vol. 27, no. 6, 1 December 2016 (2016-12-01), pages 209 - 218, XP055389324, ISSN: 1946-6536, DOI: 10.1089/hgtb.2016.120
- KATSUYUKI DODO ET AL: "An Efficient Large-Scale Retroviral Transduction Method Involving Preloading the Vector into a RetroNectin-Coated Bag with Low-Temperature Shaking", PLOS ONE, vol. 9, no. 1, 15 January 2014 (2014-01-15), pages e86275, XP055109271, ISSN: 1932-6203, DOI: 10.1371/journal.pone.0086275
- ANDREW R. MEDVEC ET AL: "Improved Expansion and In Vivo Function of Patient T Cells by a Serum-free Medium", MOLECULAR THERAPY- METHODS & CLINICAL DEVELOPMENT, vol. 8, 1 March 2018 (2018-03-01), GB, pages 65 - 74, XP055549838, ISSN: 2329-0501, DOI: 10.1016/j.omtm.2017.11.001
- E COSTELLO ET AL: "Gene transfer into stimulated and unstimulated T lymphocytes by HIV-1-derived lentiviral vectors", GENE THERAPY, vol. 7, no. 7, 1 April 2000 (2000-04-01), GB, pages 596 - 604, XP055455146, ISSN: 0969-7128, DOI: 10.1038/sj.gt.3301135
- BEYER, M ET AL.: "CD 4+ CD 25highFOXP3+ Regulatory T Cells In Peripheral Blood Are Primarily Of Effector Memory Phenotype.", JCO., vol. 25, no. 18, 20 June 2007 (2007-06-20), pages 2628 - 263, XP055237239, ISSN: 0732-183x
- KOCHENDERFER, JN ET AL.: "Construction And Pre-Clinical Evaluation Of An Anti- CD 19 Chimeric Antigen Receptor.", J IMMUNOTHER., vol. 32, no. 7, September 2009 (2009-09-01), pages 689 - 702, XP009133189

## Description

### STATEMENT OF GOVERNMENT INTEREST

This invention was created in the performance of a Cooperative Research and Development Agreement with the National Cancer Institute (NCI), an Agency of the Department of Health and Human Services. The Government of the United States has certain rights in this invention.

### BACKGROUND

The process for producing autologous engineered T cells for use in cancer therapy is lengthy (10-24 days), involves two cycles of retroviral transduction, and is poorly suited for commercial applications (see Kochenderfer et al., Blood. 2012 119: 2709-2720; Johnson, et al., Blood. 2009; 114(3):535-546). Therefore, it would be desirable to develop improvements to the T cell manufacturing process to overcome these limitations.

### SUMMARY

Provided herein is a method for manufacturing T cells which express a cell surface receptor that recognizes a specific antigenic moiety on the surface of a target cell, the method comprising (a) enriching a population of lymphocytes obtained from a donor subject; (b) stimulating the population of lymphocytes with one or more T-cell stimulating agents to produce a population of activated T cells, wherein the stimulation is performed in a closed system using serum-free culture medium; (c) transducing the population of activated T cells with a viral vector comprising a nucleic acid molecule which encodes the cell surface receptor, using a single cycle transduction to produce a population of transduced T cells, wherein the transduction is performed in a closed bag system using serum-free culture medium, wherein the bag is coated with a recombinant human fibronectin protein or fragment thereof; and (d) expanding the population of transduced T cells for a predetermined time to produce a population of engineered T cells, wherein the expansion is performed in a closed system using serum-free culture medium. In certain embodiments, the cell surface receptor may be a T cell receptor (TCR) or a chimeric antigen receptor (CAR). In certain embodiments, the target cell may be a cancer cell. In certain embodiments, the cancer cell may be a B cell malignancy. In certain embodiments, the cell surface receptor may be an anti-CD19 CAR. In certain embodiments, the anti-CD19 CAR may be a FMC63-28Z CAR or a FMC63-CD828BBz CAR. In certain embodiments, the one or more T-cell stimulating agents may be an anti-CD3 antibody and IL-2. In certain embodiments, the viral vector may be a retroviral vector. In certain embodiments, the retroviral vector may be an MSGV1 gamma retroviral vector. In certain embodiments, the MSGV1 gamma retroviral vector may be a MSGV-FMC63-28Z or a MSGV-FMC63-CD828BBz gamma retroviral vector. In certain embodiments, the predetermined time for expanding the population of transduced T cells may be 3 days. In certain embodiments, the time from enriching the population of lymphocytes to producing the engineered T cells may be 6 days. In certain embodiments, the engineered T cells may be used to treat a cancer patient. In certain embodiments, the cancer patient and the donor subject may be the same individual. In certain embodiments, the closed system may be a closed bag system. In certain embodiments, the population of cells may comprise naive T cells. In certain embodiments, about 35-43% of the population of engineered T cells may comprise naive T cells. In certain embodiments, at least about 35% of the population of engineered T cells may comprise naive T cells. In certain embodiments, at least about 43% of the population of engineered T cells may comprise naive T cells.

Described herein is a population of engineered T cells that express a cell surface receptor that recognizes a specific antigenic moiety on the surface of a target cell produced by the methods disclosed herein

Described herein are pharmaceutical Described herein compositions comprising a population of engineered T cells. Described herein are pharmaceutical compositions comprising the population of engineered T cells as described herein. The pharmaceutical composition may comprise a therapeutically effective dose of the engineered T cells. The cell surface receptor may be a T cell receptor (TCR) or a chimeric antigen receptor (CAR). The CAR may be a FMC63-28Z CAR or a FMC63-CD828BBZ CAR. The therapeutically effective dose may be more than about 1 million to less than about 3 million engineered T cells per kilogram of body weight (cells/kg). The therapeutically effective dose may be about 2 million engineered T cells/kg.

### BRIEF DESCRIPTION OF THE DRAWINGS

FIG. 1 is a diagram illustrating the T cell manufacturing process according to certain embodiments described herein (the "improved" process). Since doubling times of the T cells may vary slightly from subject to subject, additional growth time beyond 72 hours (i.e., 3-6 days) in bags is considered in the event that the total cell number is insufficient to deliver a target dose of interest (see *).
FIG. 2 is a diagram illustrating the improved process as compared to a traditionally used process (the "previous" process) according to one embodiment.

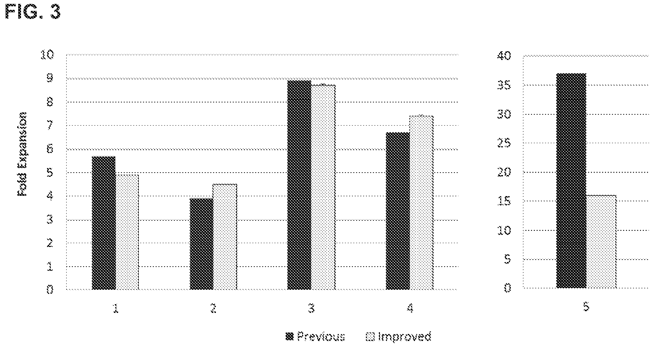

FIG. 3 is a bar graph illustrating culture expansion in the improved process as compared to the previous process according to one embodiment. The y axis shows the fold expansion of cells for each of the 5 runs (x axis). Fold culture expansion is similar between the previous and improved processes in at-scale engineering runs.
FIG. 4 shows a series of graphs illustrating T cell phenotypes on Day 6 and Day 10 in the previous and improved processes for CD3+ Cell Phenotype (FIG. 4A) and CD3+ Cell Activation (FIG. 4B) markers as shown, according to one embodiment. The T cell phenotypes are comparable between the previous and improved processes, but Day 6 cells are less differentiated. Teff= effector T cells; Tem= effector memory T cells, Tcm= central memory T cells.
FIG. 5 shows a series of graphs illustrating cell phenotype at Day 6 in the previous and improved processes, according to one embodiment.
FIG. 6 is a schematic which shows daily cell count during stimulation, transduction and expansion phases of the improved process, according to one embodiment.

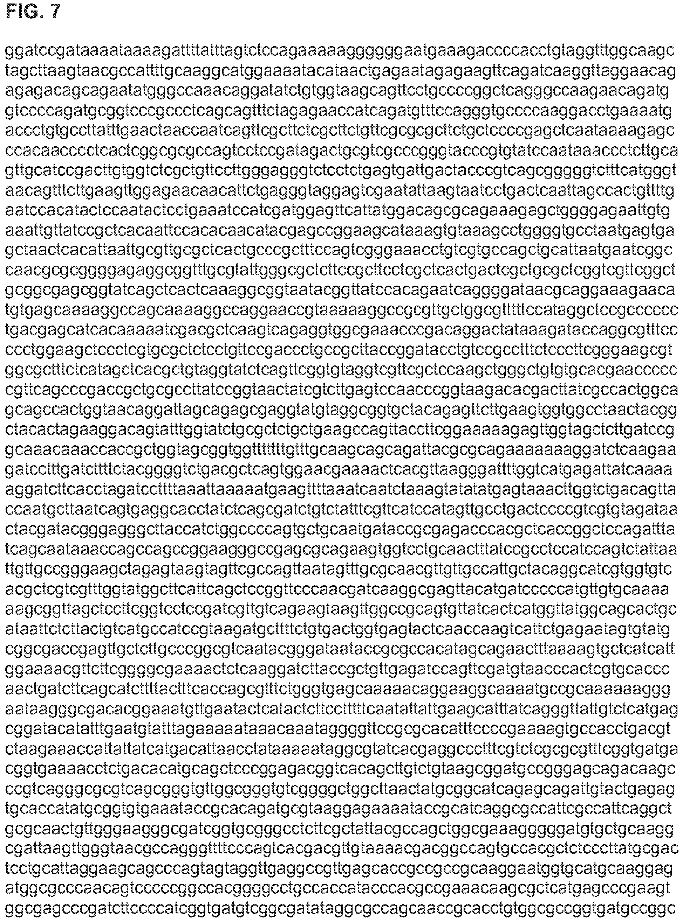

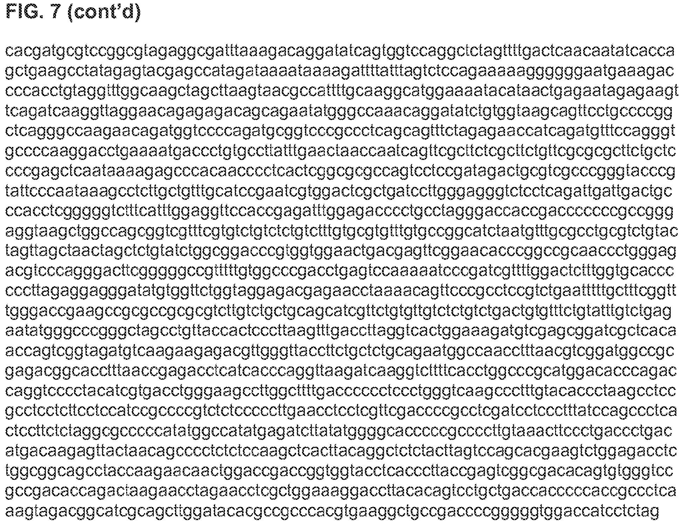

FIG. 7 shows the nucleic acid sequence of a MSGV1 gamma retroviral backbone (SEQ ID NO:4) according to one embodiment.
FIG. 8 shows the transduction efficiency as a function of RetroNectin^{®} concentration used to coat bags, according to one embodiment. RN= RetroNectin^{®} concentration in µg/mL. Results were measured on day 6 after transduction in PL07 bags from 2 donors.
FIG. 9 shows the transduction efficiency with and without wash step, according to one embodiment. Results were measured on day 6 after transduction in Origen PermaLife^{™} bags.
FIG. 10 shows the impact of RetroNectin^{®} concentration on transduction efficiency in OpTmizer^{™} medium, according to one embodiment. RN= RetroNectin^{®} concentration in µg/mL. "Open" indicates the condition where transduction was executed in plates in AIM V^{®}+ 5% human serum.
FIG. 11 shows the activity of transduced T cells as measured by CD107a expression and IFN-gamma expression after co-incubation with CD19+ Nalm6 cells for 4 hours, evaluated by FACS, according to one embodiment. "Open" indicates the condition where transduction was executed in plates in AIM V^{®}+ 5% human serum. Control T indicates a reference sample of frozen CAR-positive transduced PBMCs.
FIG. 12 shows the temperature profile of controlled rate freezer chamber (lower line) and product temperature (upper line) for the optimized profile. The displayed profile has been truncated for brevity to show only the critical region.

### DETAILED DESCRIPTION

A method for manufacturing T cell preparations is provided which may be useful for treating patients with a pathological disease or condition. In contrast to known production methods for T cell products, the methods and processes described herein are completed in a significantly shorter time, approximately 6 days, thereby offering a significant time advantage to bring the cells into the clinic. Described herein are populations of engineered T cells produced using the methods described herein, and pharmaceutical compositions thereof.

The method provided herein is used to manufacture T cells which express a cell surface receptor that recognizes a specific antigenic moiety on the surface of a target cell. The cell surface receptor may be a wild type or recombinant T cell receptor (TCR), a chimeric antigen receptor (CAR), or any other surface receptor capable of recognizing an antigenic moiety that is associated with the target cell. The form of the antigenic moiety recognized by CARs and TCRs is slightly different. CARs have a single-chain variable fragment (scFv) as a target binding domain, which allows the expression of the CAR as a single-chain protein. This allows a CAR to recognize native cancer antigens that are part of an intact protein on the target cell surface. A TCR has two protein chains, which are designed to bind with specific peptides presented by an MHC protein on the surface of certain cells. Since TCRs recognize peptides in the context of MHC molecules expressed on the surface of a target cell, TCRs have the potential to recognize cancer antigens not only presented directly on the surface of cancer cells but also presented by antigen-presenting cells in tumor, inflammatory and infected microenvironments, and in secondary lymphoid organs. Antigen-presenting cells are native immune-system cells responsible for the amplification of the immune response.

Thus, the manufactured T cells expressing the cell surface receptor may be used to target and kill any target cell, including, but not limited to, infected cells, damaged cells, or dysfunctional cells. Examples of such target cells may include cancer cells, virally infected cells, bacterially infected cells, dysfunctionally activated inflammatory cells (e.g., inflammatory endothelial cells), and cells involved in dysfunctional immune reactions (e.g., cells involved in autoimmune diseases).

In some aspects, the antigenic moiety is associated with a cancer or a cancer cell. Such antigenic moieties may include, but are not limited to, 707-AP (707 alanine proline), AFP (alpha (a)-fetoprotein), ART-4 (adenocarcinoma antigen recognized by T4 cells), BAGE (B antigen; b-catenin/m, b-catenin/mutated), BCMA (B cell maturation antigen), Bcr-abl (breakpoint cluster region-Abelson), CAIX (carbonic anhydrase IX), CD19 (cluster of differentiation 19), CD20 (cluster of differentiation 20), CD22 (cluster of differentiation 22), CD30 (cluster of differentiation 30), CD33 (cluster of differentiation 33), CD44v7/8 (cluster of differentiation 44, exons 7/8), CAMEL (CTL-recognized antigen on melanoma), CAP-1 (carcinoembryonic antigen peptide - 1), CASP-8 (caspase-8), CDC27m (cell-division cycle 27 mutated), CDK4/m (cycline-dependent kinase 4 mutated), CEA (carcinoembryonic antigen), CT (cancer/testis (antigen)), Cyp-B (cyclophilin B), DAM (differentiation antigen melanoma), EGFR (epidermal growth factor receptor), EGFRvIII (epidermal growth factor receptor, variant III), EGP-2 (epithelial glycoprotein 2), EGP-40 (epithelial glycoprotein 40), Erbb2, 3, 4 (erythroblastic leukemia viral oncogene homolog-2, -3, 4), ELF2M (elongation factor 2 mutated), ETV6-AML1 (Ets variant gene 6/acute myeloid leukemia 1 gene ETS), FBP (folate binding protein), fAchR (Fetal acetylcholine receptor), G250 (glycoprotein 250), GAGE (G antigen), GD2 (disialoganglioside 2), GD3 (disialoganglioside 3), GnT-V (N-acetylglucosaminyltransferase V), Gp100 (glycoprotein 100kD), HAGE (helicose antigen), HER-2/neu (human epidermal receptor-2/neurological; also known as EGFR2), HLA-A (human leukocyte antigen-A) HPV (human papilloma virus), HSP70-2M (heat shock protein 70 - 2 mutated), HST-2 (human signet ring tumor - 2), hTERT or hTRT (human telomerase reverse transcriptase), iCE (intestinal carboxyl esterase), IL-13R-a2 (Interleukin-13 receptor subunit alpha-2), KIAA0205, KDR (kinase insert domain receptor), κ-light chain, LAGE (L antigen), LDLR/FUT (low density lipid receptor/GDP-L-fucose: b-D-galactosidase 2-a-Lfucosyltransferase), LeY (Lewis-Y antibody), L1CAM (L1 cell adhesion molecule), MAGE (melanoma antigen), MAGE-A1 (Melanoma-associated antigen 1), mesothelin, Murine CMV infected cells, MART-1/Melan-A (melanoma antigen recognized by T cells-1/Melanoma antigen A), MC1R (melanocortin 1 receptor), Myosin/m (myosin mutated), MUC1 (mucin 1), MUM-1, -2, -3 (melanoma ubiquitous mutated 1, 2, 3), NA88-A (NA cDNA clone of patient M88), NKG2D (Natural killer group 2, member D) ligands, NY-BR-1 (New York breast differentiation antigen 1), NY-ESO-1 (New York esophageal squamous cell carcinoma-1), oncofetal antigen (h5T4), P15 (protein 15), p190 minor bcr-abl (protein of 190KD bcr-abl), Pml/RARa (promyelocytic leukaemia/retinoic acid receptor a), PRAME (preferentially expressed antigen of melanoma), PSA (prostate-specific antigen), PSCA (Prostate stem cell antigen), PSMA (prostate-specific membrane antigen), RAGE (renal antigen), RU1 or RU2 (renal ubiquitous 1 or 2), SAGE (sarcoma antigen), SART-1 or SART-3 (squamous antigen rejecting tumor 1 or 3), SSX1, -2, -3, 4 (synovial sarcoma X1, -2, -3, -4), TAA (tumor-associated antigen), TAG-72 (Tumor-associated glycoprotein 72), TEL/AML1 (translocation Ets-family leukemia/acute myeloid leukemia 1), TPI/m (triosephosphate isomerase mutated), TRP-1 (tyrosinase related protein 1, or gp75), TRP-2 (tyrosinase related protein 2), TRP-2/INT2 (TRP-2/intron 2), VEGF-R2 (vascular endothelial growth factor receptor 2), or WT1 (Wilms' tumor gene).

In some aspects, the cell surface receptor is any TCR that recognizes a specific antigenic moiety on cancer cells, including, but not limited to, an anti-707-AP TCR, anti-AFP TCR, anti-ART-4 TCR, anti-BAGE TCR, anti-Bcr-abl TCR, anti-CAMEL TCR, anti-CAP-1 TCR, anti-CASP-8 TCR, anti-CDC27m TCR, anti-CDK4/m TCR, anti-CEA TCR, anti-CT TCR, anti-Cyp-B TCR, anti-DAM TCR, anti- TCR, anti-EGFRvIII TCR, anti-ELF2M TCR, anti-ETV6-AML1 TCR, anti-G250 TCR, GAGE TCR, anti-GnT-V TCR, anti-Gp100 TCR, anti-HAGE TCR, anti-HER-2/neu TCR, anti-HLA-A TCR, anti-HPV TCR, anti-HSP70-2M TCR, anti-HST-2 TCR, anti-hTERT TCR or anti-hTRT TCR, anti-iCE TCR, anti-KIAA0205, anti-LAGE (L antigen), anti-LDLR/FUT TCR, anti-MAGE TCR, anti-MART-1/Melan-A TCR, anti-MC1R TCR, anti-Myosin/m TCR, anti-MUC1 TCR, anti-MUM-1, -2, -3 TCR, anti-NA88-A TCR, anti-NY-ESO-1 TCR, anti-P15 TCR, anti-p190 minor bcr-abl TCR, anti-Pml/RARa TCR, anti-PRAME TCR, anti-PSA TCR, anti-PSMA TCR, anti-RAGE TCR, anti-RU1 TCR or anti-RU2 TCR, anti-SAGE TCR, anti-SART-1 TCR or anti-SART-3 TCR, anti-SSX1, -2, -3, 4 TCR, anti-TEL/AML1 TCR, anti-TPI/m TCR, anti-TRP-1 TCR, anti-TRP-2 TCR, anti-TRP-2/INT2 TCR, or anti-WT1 TCR.

In other aspects, the cell surface receptor is any CAR that can be expressed by a T cell and that recognizes a specific antigenic moiety on cancer cells. Certain CARs contain an antigen binding domain (e.g., scFv) and a signaling domain (e.g., CD3 zeta chain). Other CARs contain an antigen binding domain (e.g., scFv), a signaling domain (e.g., CD3 zeta chain), and a co-stimulatory domain (e.g., CD28). Still other CARs contain an antigen binding domain (e.g., scFv), a signaling domain (e.g., CD3 zeta chain), and two co-stimulatory domains (e.g., CD28 and 4-1BB). Examples of surface receptor CARs that may be expressed by T cells that are generated in accordance with the methods described herein, include, but are not limited to, an anti-BCMA CAR, anti-CAIX CAR, anti-CD19 CAR, anti-CD20 CAR, anti-CD22 CAR, anti-CD30 CAR, anti-CD33 CAR, anti-CD44v7/8 CAR, anti-CEA CAR, anti-EGFRvlll, anti-EGP-2, anti-EGP-40 CAR, anti-Erbb2, 3, 4 CAR, anti-FBP CAR, anti-fAchR CAR, anti-GD2 CAR, anti-GD3 CAR, anti-HER2/neu CAR, anti-IL-13R-a2 CAR, anti-KDR CAR, anti-κ-light chain CAR, anti-LeY CAR, anti-L1CAM CAR, anti-MAGE-A1 CAR, anti-mesothelin CAR, CAR directed to anti-murine CMV infected cells, anti-MUC1 CAR, anti-NKG2D ligand CAR, anti-NY-BR-1 CAR, anti-h5T4 CAR, anti-PSCA CAR, anti-PSMA CAR, anti-TAA CAR, anti-TAG-72 CAR, or anti-VEGF-R2 CAR. In one embodiment, the cell surface receptor is any anti-CD19 CAR. In one aspect the anti-CD19 CAR includes an extracellular scFv domain, an intracellular and/or transmembrane, portion of a CD28 molecule, an optional extracellular portion of the CD28 molecule, and an intracellular CD3zeta domain. The anti-CD19 CAR may also include additional domains, such as a CD8 extracellular and/or transmembrane region, an extracellular immunoglobulin Fc domain (e.g., IgG1, IgG2, IgG3, IgG4), or one or more additional signaling domains, such as 41BB, OX40, CD2 CD16, CD27, CD30 CD40, PD-1, ICOS, LFA-1, IL-2 Receptor, Fc gamma receptor, or any other costimulatory domains with immunoreceptor tyrosine-based activation motifs.

In certain embodiments, the cell surface receptor is an anti-CD19 CAR, such as FMC63-28Z CAR or FMC63-CD828BBZ CAR as set forth in Kochenderfer et al., J Immunother. 2009 September; 32(7): 689-702, "Construction and Pre-clinical Evaluation of an Anti-CD19 Chimeric Antigen Receptor," which describes the methods of constructing the vectors used to produce T cells expressing the FMC63-28Z CAR or FMC63-CD828BBZ CAR.

In other embodiments, the antigenic moiety is associated with virally infected cells (i.e., a viral antigenic moiety). Such antigenic moieties may include, but are not limited to, an Epstein-Barr virus (EBV) antigen (e.g., EBNA-1, EBNA-2, EBNA-3, LMP-1, LMP-2), a hepatitis A virus antigen (e.g., VP1, VP2, VP3), a hepatitis B virus antigen (e.g., HBsAg, HBcAg, HBeAg), a hepatitis C viral antigen (e.g., envelope glycoproteins E1 and E2), a herpes simplex virus type 1, type 2, or type 8 (HSV1, HSV2, or HSV8) viral antigen (e.g., glycoproteins gB, gC, gC, gE, gG, gH, gl, gJ, gK, gL. gM, UL20, UL32, US43, UL45, UL49A), a cytomegalovirus (CMV) viral antigen (e.g., glycoproteins gB, gC, gC, gE, gG, gH, gl, gJ, gK, gL. gM or other envelope proteins), a human immunodeficiency virus (HIV) viral antigen (glycoproteins gp120, gp41, or p24), an influenza viral antigen (e.g., hemagglutinin (HA) or neuraminidase (NA)), a measles or mumps viral antigen, a human papillomavirus (HPV) viral antigen (e.g., L1, L2), a parainfluenza virus viral antigen, a rubella virus viral antigen, a respiratory syncytial virus (RSV) viral antigen, or a varicella-zostser virus viral antigen. In such aspects, the cell surface receptor may be any TCR, or any CAR which recognizes any of the aforementioned viral antigens on a target virally infected cell.

In other embodiments, the antigenic moiety is associated with cells having an immune or inflammatory dysfunction. Such antigenic moieties may include, but are not limited to, myelin basic protein (MBP) myelin proteolipid protein (PLP), myelin oligodendrocyte glycoprotein (MOG), carcinoembryonic antigen (CEA), pro-insulin, glutamine decarboxylase (GAD65, GAD67), heat shock proteins (HSPs), or any other tissue specific antigen that is involved in or associated with a pathogenic autoimmune process.

The method provided herein includes a step of enriching a population of lymphocytes obtained from a donor subject. The donor subject may be a cancer patient that is to be treated with a population of cells generated by the methods described herein (i.e., an autologous donor), or may be an individual that donates a lymphocyte sample that, upon generation of the population of cells generated by the methods described herein, will be used to treat a different individual or cancer patient (i.e., an allogeneic donor). The population of lymphocytes may be obtained from the donor subject by any suitable method used in the art. For example, the population of lymphocytes may be obtained by any suitable extracorporeal method, venipuncture, or other blood collection method by which a sample of blood and/or lymphocytes is obtained. In one embodiment, the population of lymphocytes is obtained by apheresis.

Enrichment of a population of lymphocytes may be accomplished by any suitable separation method including, but not limited to, the use of a separation medium (e.g., Ficoll-Paque^{™}, RosetteSep^{™} HLA Total Lymphocyte enrichment cocktail, Lymphocyte Separation Medium (LSA) (MP Biomedical Cat. No. 0850494X), or the like), cell size, shape or density separation by filtration or elutriation, immunomagnetic separation (e.g., magnetic-activated cell sorting system, MACS), fluorescent separation (e.g., fluorescence activated cell sorting system, FACS), or bead based column separation.

The method provided herein also includes a step of stimulating the population of lymphocytes with one or more T-cell stimulating agents to produce a population of activated T cells. Any combination of one or more suitable T-cell stimulating agents may be used to produce a population of activated T cells including, but is not limited to, an antibody or functional fragment thereof which targets a T-cell stimulatory or co-stimulatory molecule (e.g., anti-CD2 antibody, anti-CD3 antibody, anti-CD28 antibody, or functional fragments thereof) a T cell cytokine (e.g., any isolated, wildtype, or recombinant cytokines such as: interleukin 1 (IL-1), interleukin 2, (IL-2), interleukin 4 (IL-4), interleukin 5 (IL-5), interleukin 7 (IL-7), interleukin 15 (IL-15), tumor necrosis factor α (TNFa)), or any other suitable mitogen (e.g., tetradecanoyl phorbol acetate (TPA), phytohaemagglutinin (PHA), concanavalin A (conA), lipopolysaccharide (LPS), pokeweed mitogen (PWM)) or natural ligand to a T-cell stimulatory or co-stimulatory molecule.

In some embodiments, the step of stimulating the population of lymphocytes as described herein may comprise stimulating the population of lymphocytes with one or more T-cell stimulating agents at a predetermined temperature, for a predetermined amount of time, and/or in the presence of a predetermined level of CO₂. In certain embodiments, the predetermined temperature for stimulation may be about 34 °C, about 35 °C, about 36 °C, about 37 °C, about 38 °C, or about 39 °C. In certain embodiments, the predetermined temperature for stimulation may be about 34-39 °C. In certain embodiments, the predetermined temperature for stimulation may be from about 35-37 °C. In certain embodiments, the preferred predetermined temperature for stimulation may be from about 36-38 °C. In certain embodiments, the predetermined temperature for stimulation may be about 36-37 °C or more preferably about 37 °C. In certain embodiments, the step of stimulating the population of lymphocytes comprises stimulating the population of lymphocytes with one or more T-cell stimulating agents for a predetermined time. In certain embodiments, the predetermined time for stimulation may be about 24-72 hours. In certain embodiments, the predetermined time for stimulation may be about 24-36 hours, about 30-42 hours, about 36-48 hours, about 40-52 hours, about 42-54 hours, about 44-56 hours, about 46-58 hours, about 48-60 hours, about 54-66 hours, or about 60-72 hours. In certain embodiments, the predetermined time for stimulation may be about 48 hours or at least about 48 hours. In certain embodiments, the predetermined time for stimulation may be about 44-52 hours. In certain embodiments, the predetermined time for stimulation may be about 40-44 hours, about 40-48 hours, about 40-52 hours, or about 40-56 hours. In certain embodiments, the step of stimulating the population of lymphocytes may comprise stimulating the population of lymphocytes with one or more T-cell stimulating agents in the presence of a predetermined level of CO₂. In certain embodiments, the predetermined level of CO₂ for stimulation may be about 1.0-10% CO₂. In certain embodiments, the predetermined level of CO₂ for stimulation may be about 1.0%, about 2.0%, about 3.0%, about 4.0%, about 5.0%, about 6.0%, about 7.0%, about 8.0%, about 9.0%, or about 10.0% CO₂. In certain embodiments, the predetermined level of CO₂ for stimulation may be about 3-7% CO₂. In certain embodiments, the predetermined level of CO₂ for stimulation may be about 4-6% CO_{2..} In certain embodiments, the predetermined level of CO₂ for stimulation may be about 4.5-5.5% CO₂. In certain embodiments, the predetermined level of CO₂ for stimulation may be about 5% CO₂. In some embodiments, the step of stimulating the population of lymphocytes may comprise stimulating the population of lymphocytes with one or more T-cell stimulating agents at a predetermined temperature, for a predetermined amount of time, and/or in the presence of a predetermined level of CO₂ in any combination. For example, the step of stimulating the population of lymphocytes may comprise stimulating the population of lymphocytes with one or more T-cell stimulating agents at a predetermined temperature of about 36-38 °C, for a predetermined amount of time of about 44-52 hours, and in the presence of a predetermined level of CO₂ of about 4.5-5.5% CO₂.

In certain embodiments, the population of lymphocytes that is used for the step of stimulating the population of lymphocytes as described herein may be at a predetermined concentration of lymphocytes. In certain embodiments, the predetermined concentration of lymphocytes may be about 0.1 - 10.0 × 10⁶ cells/mL. In certain embodiments, the predetermined concentration of lymphocytes may be about 0.1 - 1.0 × 10⁶ cells/mL, 1.0 - 2.0 × 10⁶ cells/mL, about 1.0 - 3.0 × 10⁶ cells/mL, about 1.0 - 4.0 × 10⁶ cells/mL, about 1.0 - 5.0 × 10⁶ cells/mL, about 1.0 - 6.0 × 10⁶ cells/mL, about 1.0 - 7.0 × 10⁶ cells/mL, about 1.0 - 8.0 × 10⁶ cells/mL, 1.0 - 9.0 × 10⁶ cells/mL, or about 1.0 - 10.0 × 10⁶ cells/mL. In certain embodiments, the predetermined concentration of lymphocytes may be about 1.0 - 2.0 × 10⁶ cells/mL. In certain embodiments, the predetermined concentration of lymphocytes may be about 1.0 - 1.2 × 10⁶ cells/mL, about 1.0 - 1.4 × 10⁶ cells/mL, about 1.0 - 1.6 × 10⁶ cells/mL, about 1.0 - 1.8 × 10⁶ cells/mL, or about 1.0 - 2.0 × 10⁶ cells/mL. In certain embodiments, the predetermined concentration of lymphocytes may be at least about 0.1 × 10⁶ cells/mL, at least about 1.0 × 10⁶ cells/mL, at least about 1.1 × 10⁶ cells/mL, at least about 1.2 × 10⁶ cells/mL, at least about 1.3 × 10⁶ cells/mL, at least about 1.4 × 10⁶ cells/mL, at least about 1.5 × 10⁶ cells/mL, at least about 1.6 × 10⁶ cells/mL, at least about 1.7 × 10⁶ cells/mL, at least about 1.8 × 10⁶ cells/mL, at least about 1.9 × 10⁶ cells/mL, at least about 2.0 × 10⁶ cells/mL, at least about 4.0 × 10⁶ cells/mL, at least about 6.0 × 10⁶ cells/mL, at least about 8.0 × 10⁶ cells/mL, or at least about 10.0 × 10⁶ cells/mL.

In some embodiments, an anti-CD3 antibody (or functional fragment thereof), an anti-CD28 antibody (or functional fragment thereof), or a combination of anti-CD3 and anti-CD28 antibodies may be used in accordance with the step of stimulating the population of lymphocytes. Any soluble or immobilized anti-CD2, anti-CD3 and/or anti-CD28 antibody or functional fragment thereof may be used (e.g., clone OKT3 (anti-CD3), clone 145-2C11 (anti-CD3), clone UCHT1 (anti-CD3), clone L293 (anti-CD28), clone 15E8 (anti-CD28)). In some aspects, the antibodies may be purchased commercially from vendors known in the art including, but not limited to, Miltenyi Biotec, BD Biosciences (e.g., MACS GMP CD3 pure 1mg/mL, Part No. 170-076-116), and eBioscience, Inc. Further, one skilled in the art would understand how to produce an anti-CD3 and/or anti-CD28 antibody by standard methods. Any antibody used in the methods described herein should be produced under Good Manufacturing Practices (GMP) to conform to relevant agency guidelines for biologic products. In some embodiments, the one or more T cell stimulating agents that may be used in accordance with the step of stimulating the population of lymphocytes include an antibody or functional fragment thereof which targets a T-cell stimulatory or co-stimulatory molecule in the presence of a T cell cytokine. In one embodiment the one or more T cell stimulating agents include an anti-CD3 antibody and IL-2. In certain embodiments, the T cell stimulating agent may include an anti-CD3 antibody at a concentration of from about 20 ng/mL-100 ng/mL. In certain embodiments, the concentration of anti-CD3 antibody may be about 20 ng/mL, about 30 ng/mL, about 40 ng/mL, about 50 ng/mL, about 60 ng/mL, about 70 ng/mL, about 80 ng/mL, about 90 ng/mL, or about 100 ng/mL. In certain embodiments, the concentration of anti-CD3 antibody may be about 50 ng/mL.

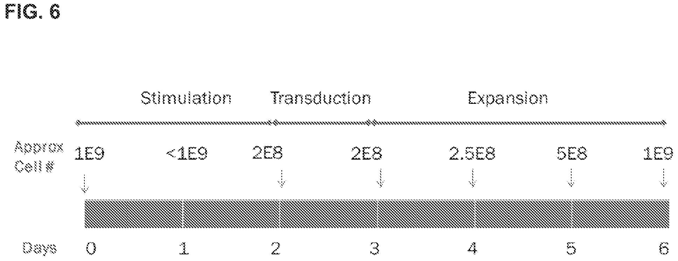

The method provided herein further includes a step of transducing the population of activated T cells with a viral vector comprising a nucleic acid molecule which encodes the cell surface receptor, using a single cycle transduction to produce a population of transduced T cells. Several recombinant viruses have been used as viral vectors to deliver genetic material to a cell. Viral vectors that may be used in accordance with the transduction step may be any ecotropic or amphotropic viral vector including, but not limited to, recombinant retroviral vectors, recombinant lentiviral vectors, recombinant adenoviral vectors, and recombinant adeno-associated viral (AAV) vectors. In one embodiment, the viral vector used to transduce the population of activated T cells is an MSGV1 gamma retroviral vector. In one aspect such an MSGV1 gamma retroviral vector may include a backbone nucleic acid sequence shown in FIG. 6 (SEQ ID NO:4), wherein a nucleic acid fragment that includes the sequence of a cell surface receptor (e.g., a CAR or a TCR) is ligated with a nucleic acid fragment that includes the sequence of the MSGV1 gamma retroviral vector. In certain embodiments, the viral vector used to transduce the population of activated T cells may be the MSGV-FMC63-28Z retroviral vector or the MSGV-FMC63-CD828BBZ retroviral vector as set forth in Kochenderfer et al., J Immunother. 2009 September; 32(7): 689-702, which describes the methods of constructing the retroviral vectors as provided in the "Construction of the MSGV-FMC63-28Z and MSGV-FMC63-CD828BBZ Recombinant Retroviral Vectors" section in the "Materials and Methods" section of the publication. According to one aspect of this embodiment, the viral vector is grown in a culture in a medium which is specific for viral vector manufacturing. Any suitable growth media and/or supplements for growing viral vectors may be used in the viral vector inoculum in accordance with the methods described herein. According to some aspects, the viral vector may then be added to the serum-free culture media described below during the transduction step.

In certain embodiments, the step of transducing the population of activated T cells may be performed for a predetermined time, at a predetermined temperature and/or in the presence of a predetermined level of CO₂. In certain embodiments, the predetermined temperature for transduction may be about 34 °C, about 35 °C, about 36 °C, about 37 °C, about 38 °C, or about 39 °C. In certain embodiments, the predetermined temperature for transduction may be about 34-39 °C. In certain embodiments, the predetermined temperature for transduction may be from about 35-37 °C. In certain embodiments, the preferred predetermined temperature for transduction may be from about 36-38 °C. In certain embodiments, the predetermined temperature for transduction may be about 36-37 °C or more preferably about 37 °C. In certain embodiments, the predetermined time for transduction may be about 12-36 hours. In certain embodiments, the predetermined time for transduction may be about 12-16 hours, about 12-20 hours, about 12-24 hours, about 12-28 hours, or about 12-32 hours. In certain embodiments, the predetermined time for transduction may be about 20 hours or at least about 20 hours. In certain embodiments, the predetermined time for transduction may be about 16-24 hours. In certain embodiments, the predetermined time for transduction may be at least about 14 hours, at least about 16 hours, at least about 18 hours, at least about 20 hours, at least about 22 hours, at least about 24 hours, or at least about 26 hours. In some embodiments, the step of transducing the population of activated T cells may comprise transducing the population of activated T cells with a viral vector at a predetermined level of CO₂. In certain embodiments, the predetermined level of CO₂ for transduction may be about 1.0-10% CO₂. In certain embodiments, the predetermined level of CO₂ for transduction may be about 1.0%, about 2.0%, about 3.0%, about 4.0%, about 5.0%, about 6.0%, about 7.0%, about 8.0%, about 9.0%, or about 10.0% CO₂. In certain embodiments, the predetermined level of CO₂ for transduction may be about 3-7% CO₂. In certain embodiments, the predetermined level of CO₂ for transduction may be about 4-6% CO_{2..} In certain embodiments, the predetermined level of CO₂ for transduction may be about 4.5-5.5% CO₂. In certain embodiments, the predetermined level of CO₂ for transduction may be about 5% CO₂. In some embodiments, the step of transducing the population of activated T cells may be performed for a predetermined time, at a predetermined temperature and/or in the presence of a predetermined level of CO₂ in any combination. For example, the step of transducing the population of activated T cells may comprise a predetermined temperature of about 36-38 °C, for a predetermined amount of time of about 16-24 hours, and in the presence of a predetermined level of CO₂ of about 4.5-5.5% CO₂.

The method provided herein further includes a step of expanding the population of transduced T cells for a predetermined time to produce a population of engineered T cells. The predetermined time for expansion may be any suitable time which allows for the production of (i) a sufficient number of cells in the population of engineered T cells for at least one dose for administering to a patient, (ii) a population of engineered T cells with a favorable proportion of juvenile cells compared to a typical longer process, or (iii) both (i) and (ii). This time will depend on the cell surface receptor expressed by the T cells, the vector used, the dose that is needed to have a therapeutic effect, and other variables. Thus, in some embodiments, the predetermined time for expansion may be 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, or more than 21 days. In some aspects, the predetermined time for expansion is shorter than expansion methods known in the art. For example, the predetermined time for expansion may be shorter by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or may be shorter by more than 75%. In one embodiment, the predetermined time for expansion is about 3 days. In this aspect, the time from enrichment of the population of lymphocytes to producing the engineered T cells is about 6 days. In certain embodiments, the step of expanding the population of transduced T cells may be performed at a predetermined temperature and/or in the presence of a predetermined level of CO₂. In certain embodiments, the predetermined temperature may be about 34 °C, about 35 °C, about 36 °C, about 37 °C, about 38 °C, or about 39 °C. In certain embodiments, the predetermined temperature may be about 34-39 °C. In certain embodiments, the predetermined temperature may be from about 35-37 °C. In certain embodiments, the preferred predetermined temperature may be from about 36-38 °C. In certain embodiments, the predetermined temperature may be about 36-37 °C or more preferably about 37 °C. In some embodiments, step of expanding the population of transduced T cells may comprise expanding the population of transduced T cells in the presence of a predetermined level of CO₂. In certain embodiments, the predetermined level of CO₂ may be 1.0-10% CO₂. In certain embodiments, the predetermined level of CO₂ may be about 1.0%, about 2.0%, about 3.0%, about 4.0%, about 5.0%, about 6.0%, about 7.0%, about 8.0%, about 9.0%, or about 10.0% CO₂. In certain embodiments, the predetermined level of CO₂ may be about 4.5-5.5% CO₂. In certain embodiments, the predetermined level of CO₂ may be about 5% CO₂. In certain embodiments, the predetermined level of CO₂ may be about 3.5%, about 4.0%, about 4.5%,about 5.0%, about 5.5%, or about 6.5% CO₂. In some embodiments, the step of expanding the population of transduced T cells may be performed at a predetermined temperature and/or in the presence of a predetermined level of CO₂ in any combination. For example, the step of expanding the population of transduced T cells may comprise a predetermined temperature of about 36-38 °C and in the presence of a predetermined level of CO₂ of about 4.5-5.5% CO₂.

In some aspects, each step of the methods described herein is performed in a closed system. In certain embodiments, the closed system is a closed bag culture system, which may use any suitable cell culture bags (e.g., Mitenyi Biotec MACS^{®} GMP Cell Differentiation Bags, Origen Biomedical PermaLife^{™} Cell Culture bags). In some embodiments, the cell culture bags used in the closed bag culture system are coated with a recombinant human fibronectin protein during the transduction step. In certain embodiments, the cell culture bags used in the closed bag culture system are coated with a recombinant human fibronectin protein fragment during the transduction step. The recombinant human fibronectin fragment may include three functional domains: a central cell-binding domain, heparin-binding domain II, and a CS1-sequence. The recombinant human fibronectin protein or fragment thereof may be used to increase gene efficiency of retroviral transduction of immune cells by aiding co-localization of target cells and viral vector. In certain embodiments, the recombinant human fibronectin fragment is RetroNectin^{®} (Takara Bio, Japan). In certain embodiments, the cell culture bags may be coated with recombinant human fibronectin fragment at a concentration of about 1-60 µg/mL, preferably 1-40 µg/mL. In certain embodiments, the cell culture bags may be coated with recombinant human fibronectin fragment at a concentration of about 1-20 µg/mL, 20-40 µg/mL, or 40-60 µg/mL. In certain embodiments, the cell culture bags may be coated with about 1 µg/mL, about 2 µg/mL, about 3 µg/mL, about 4 µg/mL, about 5 µg/mL, about 6 µg/mL, about 7 µg/mL, about 8 µg/mL, about 9 µg/mL, about 10 µg/mL, about 11 µg/mL, about 12 µg/mL, about 13 µg/mL, about 14 µg/mL, about 15 µg/mL, about 16 µg/mL, about 17 µg/mL, about 18 µg/mL, about 19 µg/mL, or about 20 µg/mL recombinant human fibronectin fragment. In certain embodiments, the cell culture bags may be coated with about 2-5 µg/mL, about 2-10 µg/mL, about 2-20 µg/mL, about 2-25 µg/mL, about 2-30 µg/mL, about 2-35 µg/mL, about 2-40 µg/mL, about 2-50 µg/mL, or about 2-60 µg/mL recombinant human fibronectin fragment. In certain embodiments, the cell culture bags may be coated with at least about 2 µg/mL, at least about 5 µg/mL, at least about 10 µg/mL, at least about 15 µg/mL, at least about 20 µg/mL, at least about 25 µg/mL, at least about 30 µg/mL, at least about 40 µg/mL, at least about 50 µg/mL, or at least about 60 µg/mL recombinant human fibronectin fragment. In certain embodiments, the cell culture bags may be coated with at least about 10 µg/mL recombinant human fibronectin fragment. In certain embodiments, the cell culture bags used in the closed bag culture system may be blocked with human albumin serum (HSA) during the transduction step. In an alternative embodiment, the cell culture bags are not blocked with HSA during the transduction step. As referred to herein, the term "serum-free media" or "serum-free culture medium" means that the growth media used is not supplemented with serum (e.g., human serum or bovine serum). In other words, no serum is added to the culture medium as an individually separate and distinct ingredient for the purpose of supporting the viability, activation and grown of the cultured cells. Any suitable culture medium T cell growth media may be used for culturing the cells in suspension in accordance with the methods described herein. For example a T cell growth media may include, but is not limited to, a sterile, low glucose solution that includes a suitable amount of buffer, magnesium, calcium, sodium pyruvate, and sodium bicarbonate. In one embodiment, the T cell growth media is OpTmizer^{™} (Life Technologies), but one skilled in the art would understand how to generate similar media. In contrast to typical methods for producing engineered T cells, the methods described herein use culture medium that is not supplemented with serum (e.g., human or bovine).

The method for manufacturing T cells which express a cell surface receptor that recognizes a specific antigenic moiety on the surface of a target cell provided herein comprises (1) enriching a population of lymphocytes obtained from a donor subject; (2) stimulating the population of lymphocytes with one or more T-cell stimulating agents to produce a population of activated T cells, wherein the stimulation is performed in a closed system using a serum-free culture medium; (3) transducing the population of activated T cells with a viral vector comprising a nucleic acid molecule which encodes the cell surface receptor, using a single cycle transduction to produce a population of transduced T cells, wherein the transduction is performed in a closed bag system using a serum-free culture medium wherein the bag is coated with a recombinant human fibronectin protein or fragment thereof; and (4) expanding the population of transduced T cells for a predetermined time to produce a population of engineered T cells, wherein the expansion is performed in a closed system using a serum-free culture medium.

In one embodiment, the method may include, but is not limited to, (1) collection of apheresis product from a patient and separation of the mononuclear cells in a closed system, (2) stimulation of the mononuclear cell population with an antibody to CD3 in the presence of IL2 to stimulate cell growth of T cells in a closed system, (3) transduction of a new cell surface receptor gene that allows T cells to recognize a specific antigenic moiety on the surface of cancer target cells using a gamma retroviral vector in a closed system, (4) expansion of the transduced T cell in a closed system, (5) and wash and preparation of the expanded autologous T cells in a closed system for re-administration to a cancer patient. In some embodiments, the expansion step is 3 days, allowing for the entire manufacturing process to be completed in less than one week. Process steps 2-4 where T cells are actively growing are performed in defined cell culture medium that does not contain human serum (i.e., serum-free medium). T cells produced by this process exhibit biologic activity and become activated by target antigen on the surface of cancer cells and produce gamma interferon in response. Some aspects of the methods described herein, include:
- This process occurs in a closed system where the likelihood of contamination during T cell manufacturing is minimal;
- The process is suitable for production of T cells for clinical applications;
- Cells are propagated in a cell culture medium that does not contain human serum;
- Receptor gene is introduced into T cells in a closed bag system;
- Cells can be prepared for clinical use in only 6 days; and
- Cells exhibit biological activity indicative of biological activity *in vivo.*

These aspects provide several distinctions and/or improvements over the methods that are currently used in the field as follows. Use of human serum-free cell culture medium minimizes the opportunities for introduction of human pathogens from raw materials in the process, and avoids use of a raw material that may not be readily available in the future. In addition, such use supports GMP compliance since different serum lots require significant culture testing and release to ensure reproducibility and process robustness. T cell growth in serum-free medium has been reported previously (Carstens et al., ISCT meeting in San Diego, 2012; Zuliani, 2011), but it has not previously been incorporated into a process for production of T cells for clinical use to treat cancer, and previous work has not shown that T cell activation, transduction, and expansion in serum-free medium is robust. In the improved process contemplated in the studies described herein, the use of an anti-CD3 monoclonal antibody and IL2 were retained for stimulation of T cell populations. Further, cell culture in closed-system bags would provide a significant advantage to prevent possible contamination during cell culture and provide a simplified and shortened process that is suitable for cGMP manufacturing and product commercialization. The importance of this practical application is significant, since many processes described in the literature for T cell propagation are not suitable for widespread commercial applications.

Previously, viral transduction with a gamma retroviral vector has not been efficient, and an open process called 'spinocculation' has been performed in microtiter plates, where virus and cells were centrifuged onto the bottom of a well that had been coated with RetroNectin^{®}. This process is typically repeated twice on sequential days to maximize the transduction efficiency. This transduction step was modified in accordance with some embodiments of the methods described herein such that the transduction is performed in a closed bag system, using a bag (rather than a plate) that had been coated with RetroNectin^{®}, and that the process be executed a single time rather than twice. The methods described herein may also involve propagation of cells in closed system cell culture bags rather than open system flasks as had historically been used in the field. Although some literature includes reports of transduction in bag systems (Lamers et al., Cancer Gene Therapy 2002, 9: 613-623; Lamers et al, Cytotherapy 2008, 10: 406-416; Tumanini et al, Cytotherapy 2013, 11, 1406-1415), these cases - unlike the methods described herein - include at least two transductions that had been completed in cell culture medium that contained serum, and an expansion time of at least 9 days. The development studies described herein demonstrate that transduction in bags in serum-free medium is not only feasible, but that transduction levels are acceptable for further clinical development after a single transduction and an expansion of only 3 days.

The population of engineered T cells produced by the methods described above may optionally be cryopreserved so that the cells may be used at a later date. Thus, a method for cryopreservation of a population of engineered T cells is described herein. Such a method may include a step of washing and concentrating the population of engineered T cells with a diluent solution. In some aspects the diluent solution is normal saline, 0.9% saline, PlasmaLyte A (PL), 5% dextrose/0.45% NaCl saline solution (D5), human serum albumin (HSA), or a combination thereof. In some aspects, HSA may be added to the washed and concentrated cells for improved cell viability and cell recovery after thawing. In another aspect, the washing solution is normal saline and washed and concentrated cells are supplemented with HSA (5%). The method may also include a step of generating a cryopreservation mixture, wherein the cryopreservation mixture includes the diluted population of cells in the diluent solution and a suitable cryopreservative solution. In some aspects, the cryopreservative solution may be any suitable cryopreservative solution including, but not limited to, CryoStor10 (BioLife Solution), mixed with the diluent solution of engineered T cells at a ratio of 1:1 or 2:1. HSA may be added to provide a final concentration of about 1.0-10% HSA in the cryopreserved mixture. HSA may be added to provide a final concentration of about 1.0%, about 2.0%, about 3.0%, about 4.0%, about 5.0%, about 6.0%, about 7.0%, about 8.0%, about 9.0%, or about 10.0% HSA in the cryopreserved mixture. HSA may be added to provide a final concentration of about 1-3% HSA, about 1-4% HSA, about 1-5% HSA, about 1-7% HSA, about 2-4% HSA, about 2-5% HSA, about 2-6% HSA, or about 2-7% HSA in the cryopreserved mixture. HSA may be added to provide a final concentration of about 2.5% HSA in the cryopreserved mixture. For example, cryopreservation of a population of engineered T cells may comprise washing cells with 0.9% normal saline, adding HSA at a final concentration of 5% to the washed cells, and diluting the cells 1:1 with CryoStor^{™} CS10 (for a final concentration of 2.5% HSA in the final cryopreservation mixture). The method may also include a step of freezing the cryopreservation mixture. In one aspect, the cryopreservation mixture is frozen in a controlled rate freezer using a defined freeze cycle at a cell concentration of between about 1e6 to about 1.5e7 cells per mL of cryopreservation mixture. The method may also include a step of storing the cryopreservation mixture in vapor phase liquid nitrogen.

The population of engineered T cells produced by the methods described herein may be cryopreserved at a predetermined dose. The predetermined dose may be a therapeutically effective dose, which may be any therapeutically effective dose as described below. The predetermined dose of engineered T cells may depend on the cell surface receptor that is expressed by the T cells (e.g., the affinity and density of the cell surface receptors expressed on the cell), the type of target cell, the nature of the disease or pathological condition being treated, or a combination of both. In certain embodiments, the cell surface receptor that is expressed by the engineered T cells may be an anti-CD19 CAR, such as FMC63-28Z CAR or FMC63-CD828BBZ CAR as set forth in Kochenderfer et al., J Immunother. 2009 September; 32(7): 689-702, which describes methods of constructing the vectors used to produce T cells expressing a FMC63-28Z CAR or a FMC63-CD828BBZ CAR. The predetermined dose of engineered T cells expressing a FMC63-28Z CAR or a FMC63-CD828BBZ CAR may be more than about 1 million to less than about 3 million transduced engineered T cells/kg. The predetermined dose of engineered T cells expressing a FMC63-28Z CAR or a FMC63-CD828BBZ CAR may be more than about 1 million to about 2 million transduced engineered T cells per kilogram of body weight (cells/kg). The predetermined dose of engineered T cells expressing a FMC63-28Z CAR or a FMC63-CD828BBZ CAR may be more than 1 million to about 2 million transduced engineered T cells per kilogram of body weight (cells/kg). The predetermined dose of engineered T cells expressing a FMC63-28Z CAR or a FMC63-CD828BBZ CAR may be at least about 2 million to less than about 3 million transduced engineered T cells/kg. The preferred predetermined dose of engineered T cells expressing a FMC63-28Z CAR or a FMC63-CD828BBZ CAR may be about 2 million transduced engineered T cells/kg. The predetermined dose of engineered T cells expressing a FMC63-28Z CAR or a FMC63-CD828BBZ CAR may be at least about 2 million transduced engineered T cells/kg. The predetermined dose of engineered T cells expressing a FMC63-28Z CAR or a FMC63-CD828BBZ CAR may be about 2.0 million, about 2.1 million, about 2.2 million, about 2.3 million, about 2.4 million, about 2.5 million, about 2.6 million, about 2.7 million, about 2.8 million, or about 2.9 million transduced engineered T cells/kg. The population of engineered T cells may be cryopreserved at a predetermined dose of about 1 million engineered T cells per kilogram of body weight (cells/kg). The population of engineered T cells may be cryopreserved at a predetermined dose of from about 500,000 to about 1 million engineered T cells/kg. The population of engineered T cells may be cryopreserved at a predetermined dose of at least about 1 million, at least about 2 million, at least about 3 million, at least about 4 million, at least about 5 million, at least about 6 million, at least about 7 million, at least about 8 million, at least about 9 million, at least about 10 million engineered T cells/kg. In other aspects, the population of engineered T cells may be cryopreserved at a predetermined dose of less than 1 million cells/kg, 1 million cells/kg, 2 million cells/kg, 3 million cells/kg, 4 million cells/kg, 5 million cells/kg, 6 million cells/kg, 7 million cells/kg, 8 million cells/kg, 9 million cells/kg, 10 million cells/kg, more than 10 million cells/kg, more than 20 million cells/kg, more than 30 million cells/kg, more than 40 million cells/kg, more than 50 million cells/kg, more than 60 million cells/kg, more than 70 million cells/kg, more than 80 million cells/kg, more than 90 million cells/kg, or more than 100 million cells/kg. The population of engineered T cells may be cryopreserved at a predetermined dose of from about 1 million to about 2 million engineered T cells/kg. The population of engineered T cells may be cryopreserved at a predetermined dose between about 1 million cells to about 2 million cells/kg, about 1 million cells to about 3 million cells/kg, about 1 million cells to about 4 million cells/kg, about 1 million cells to about 5 million cells/kg, about 1 million cells to about 6 million cells/kg, about 1 million cells to about 7 million cells/kg, about 1 million cells to about 8 million cells/kg, about 1 million cells to about 9 million cells/kg, about 1 million cells to about 10 million cells/kg. The predetermined dose of the population of engineered T cells may be calculated based on a subject's body weight. The population of engineered T cells may be cryopreserved in about 0.5-200 mL of cryopreservation media. The population of engineered T cells may be cryopreserved in about 0.5 mL, about 1.0 mL, about 5.0 mL, about 10.0 mL, about 20 mL, about 30 mL, about 40 mL, about 50 mL, about 60 mL, about 70 mL, about 80 mL, about 90 mL, or about 100 mL of cryopreservation media. The population of engineered T cells may be cryopreserved in about 10-30 mL, about 10-50 mL, about 10-70 mL, about 10-90 mL, about 50-70 mL, about 50-90 mL, about 50-110 mL, about 50-150 mL, or about 100-200 mL of cryopreservation media. The population of engineered T cells may be preferably cryopreserved in about 50-70 mL of cryopreservation media.

The methods described herein are used to produce a population of engineered T cells that may be used to treat a disease or pathological condition in a subject having the disease or pathological condition by administering a therapeutically effective amount or therapeutically effective dose of the engineered T cells to the subject. As such, a population of engineered T cells that express a cell surface receptor that recognize a specific antigenic moiety on the surface of a target cell may be produced by a method provided herein. Pathogenic conditions that may be treated with engineered T cells that are produced by the methods described herein include, but are not limited to, cancer, viral infection, acute or chronic inflammation, autoimmune disease or any other immune-dysfunction. In one embodiment, the engineered T cells are for use in the treatment of a cancer patient, wherein the cancer patient and the donor subject are preferably the same individual. Examples of treating patients with doses of engineered T cells may be found in Kochenderfer, et al., J Clin Oncol. 2014 Aug 25. pii: JCO.2014.56.2025 (entitled "Chemotherapy-Refractory Diffuse Large B-Cell Lymphoma and Indolent B-Cell Malignancies Can Be Effectively Treated With Autologous T Cells Expressing an Anti-CD19 Chimeric Antigen Receptor") and Kochenderfer et al. Blood. 2012 Mar 22;119(12):2709-20 (entitled "B-cell depletion and remissions of malignancy along with cytokine-associated toxicity in a clinical trial of anti-CD19 chimeric-antigen-receptor-transduced T cells"), which provide details regarding the standard practices of treating patients with doses of engineered T cells.

The population of engineered T cells produced by the methods described above may comprise one or more subpopulations of cells. The one or more subpopulations of cells may include, without limitation, naive T cells, effector T cells, effector memory T cells, and/or central memory T cells. As provided in Example 2 below, it was unexpected that using the methods described herein, in addition to simply diminishing the duration of culture from 10 days or longer to 6 days, would result in a more juvenile T cell distribution with increased representation of naive T cells and decreased representation of differentiated effector T cells. In certain embodiments, the population of engineered T cells may comprise a subpopulation of naive T cells. In certain embodiments, at least about 34-43% of the population of engineered T cells may comprise a subpopulation of naive T cells. In certain embodiments, at least about 35% of the population of engineered T cells may comprise a subpopulation of naive T cells. In certain embodiments, at least about 40% of the population of engineered T cells may comprise a subpopulation of naive T cells. In certain embodiments, at least about 34%, at least about 35%, at least about 36%, at least about 37%, at least about 38%, at least about 39%, at least about 40%, at least about 41%, at least about 42%, at least about 43%, or at least about 44% of the population of engineered T cells may comprise a subpopulation of naive T cells. In certain embodiments, the population of engineered T cells may comprise a subpopulation of central memory T cells. In certain embodiments, about 15% or less of the population of engineered T cells may comprise a subpopulation of central memory T cells. In certain embodiments, about 15% or less, about 14% or less, about 13% or less, about 12% or less, about 11% or less of the population of engineered T cells may comprise a subpopulation of central memory T cells.

As referred to herein, a "cancer" may be any cancer that is associated with a surface antigen or cancer marker, including, but not limited to, acute lymphoblastic leukemia (ALL), acute myeloid leukemia (AML), adenoid cystic carcinoma, adrenocortical, carcinoma, AIDS-related cancers, anal cancer, appendix cancer, astrocytomas, atypical teratoid/rhabdoid tumor, central nervous system, B-cell leukemia, lymphoma or other B cell malignancies, basal cell carcinoma, bile duct cancer, bladder cancer, bone cancer, osteosarcoma and malignant fibrous histiocytoma, brain stem glioma, brain tumors, breast cancer, bronchial tumors, burkitt lymphoma, carcinoid tumors, central nervous system cancers, cervical cancer, chordoma, chronic lymphocytic leukemia (CLL), chronic myelogenous leukemia (CML), chronic myeloproliferative disorders, colon cancer, colorectal cancer, craniopharyngioma, cutaneous t-cell lymphoma, embryonal tumors, central nervous system, endometrial cancer, ependymoblastoma, ependymoma, esophageal cancer, esthesioneuroblastoma, ewing sarcoma family of tumors extracranial germ cell tumor, extragonadal germ cell tumor extrahepatic bile duct cancer, eye cancer fibrous histiocytoma of bone, malignant, and osteosarcoma, gallbladder cancer, gastric (stomach) cancer, gastrointestinal carcinoid tumor, gastrointestinal stromal tumors (GIST), soft tissue sarcoma, germ cell tumor, gestational trophoblastic tumor, glioma, hairy cell leukemia, head and neck cancer, heart cancer, hepatocellular (liver) cancer, histiocytosis, hodgkin lymphoma, hypopharyngeal cancer, intraocular melanoma, islet cell tumors (endocrine pancreas), kaposi sarcoma, kidney cancer, langerhans cell histiocytosis, laryngeal cancer, leukemia, lip and oral cavity cancer, liver cancer (primary), lobular carcinoma in situ (LCIS), lung cancer, lymphoma, macroglobulinemia, male breast cancer, malignant fibrous histiocytoma of bone and osteosarcoma, medulloblastoma, medulloepithelioma, melanoma, merkel cell carcinoma, mesothelioma, metastatic squamous neck cancer with occult primary midline tract carcinoma involving NUT gene, mouth cancer, multiple endocrine neoplasia syndromes, multiple myeloma/plasma cell neoplasm, mycosis fungoides, myelodysplastic syndromes, myelodysplastic/myeloproliferative neoplasms, myelogenous leukemia, chronic (CML), Myeloid leukemia, acute (AML), myeloma, multiple, myeloproliferative disorders, nasal cavity and paranasal sinus cancer, nasopharyngeal cancer, neuroblastoma, non-hodgkin lymphoma, non-small cell lung cancer, oral cancer, oral cavity cancer, oropharyngeal cancer, osteosarcoma and malignant fibrous histiocytoma of bone, ovarian cancer, pancreatic cancer, papillomatosis, paraganglioma, paranasal sinus and nasal cavity cancer, parathyroid cancer, penile cancer, pharyngeal cancer, pheochromocytoma, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, primary central nervous system (CNS) lymphoma, prostate cancer, rectal cancer, renal cell (kidney) cancer, renal pelvis and ureter, transitional cell cancer, retinoblastoma, rhabdomyosarcoma, salivary gland cancer, sarcoma, sézary syndrome, small cell lung cancer, small intestine cancer, soft tissue sarcoma, squamous cell carcinoma, squamous neck cancer, stomach (gastric) cancer, supratentorial primitive neuroectodermal tumors, t-cell lymphoma, cutaneous, testicular cancer, throat cancer, thymoma and thymic carcinoma, thyroid cancer, transitional cell cancer of the renal pelvis and ureter, trophoblastic tumor, ureter and renal pelvis cancer, urethral cancer, uterine cancer, uterine sarcoma, vaginal cancer, vulvar cancer, Waldenström macroglobulinemia, Wilms Tumor.

In some aspects, the cancer is a B cell malignancy. Examples of B cell malignancies include, but are not limited to, Non-Hodgkin's Lymphomas (NHL), Diffuse Large B Cell Lymphoma (DLBCL), Small lymphocytic lymphoma (SLL/CLL), Mantle cell lymphoma (MCL), Follicular lymphoma (FL), Marginal zone lymphoma (MZL), Extranodal (MALT lymphoma), Nodal (Monocytoid B-cell lymphoma), Splenic, Diffuse large cell lymphoma, B cell chronic lymphocytic leukemia/lymphoma, Burkitt's lymphoma and Lymphoblastic lymphoma.

As referred to herein, a "viral infection" may be an infection caused by any virus which causes a disease or pathological condition in the host. Examples of viral infections that may be treated with the engineered T cells that are produced by the methods described herein include, but are not limited to, a viral infection caused by an Epstein-Barr virus (EBV); a viral infection caused by a hepatitis A virus, a hepatitis B virus or a hepatitis C virus; a viral infection caused by a herpes simplex type 1 virus, a herpes simplex type 2 virus, or a herpes simplex type 8 virus, a viral infection caused by a cytomegalovirus (CMV), a viral infection caused by a human immunodeficiency virus (HIV), a viral infection caused by an influenza virus, a viral infection caused by a measles or mumps virus, a viral infection caused by a human papillomavirus (HPV), a viral infection caused by a parainfluenza virus, a viral infection caused by a rubella virus, a viral infection caused by a respiratory syncytial virus (RSV), or a viral infection caused by a varicella-zostser virus. In some aspects, a viral infection may lead to or result in the development of cancer in a subject with the viral infection (e.g., HPV infection may cause or be associated with the development of several cancers, including cervical, vulvar, vaginal, penile, anal, oropharyngeal cancers, and HIV infection may cause the development of Kaposi's sarcoma)

Examples of chronic inflammation diseases, autoimmune diseases or any other immune-dysfunctions that may be treated with the engineered T cells produced by the methods described herein include, but are not limited to, multiple sclerosis, lupus, and psoriasis.

The term "treat," "treating" or "treatment" as used herein with regard to a condition or disease may refer to preventing a condition or disease, slowing the onset or rate of development of the condition or disease, reducing the risk of developing the condition or disease, preventing or delaying the development of symptoms associated with the condition or disease, reducing or ending symptoms associated with the condition or disease, generating a complete or partial regression of the condition or disease, or some combination thereof.

A "therapeutically effective amount" or a "therapeutically effective dose" is an amount of engineered T cells that produce a desired therapeutic effect in a subject, such as preventing or treating a target condition or alleviating symptoms associated with the condition by killing target cells. The most effective results in terms of efficacy of treatment in a given subject will vary depending upon a variety of factors, including but not limited to the characteristics of the engineered T cells (including longevity, activity, pharmacokinetics, pharmacodynamics, and bioavailability), the physiological condition of the subject (including age, sex, disease type and stage, general physical condition, responsiveness to a given dosage, and type of medication), the nature of any pharmaceutically acceptable carrier or carriers in any composition used, and the route of administration. A therapeutically effective dose of engineered T cells also depends on the cell surface receptor that is expressed by the T cells (e.g., the affinity and density of the cell surface receptors expressed on the cell), the type of target cell, the nature of the disease or pathological condition being treated, or a combination of both. Therefore, in some aspects, a therapeutically effective dose of transduced engineered T cells is from about 1 million to about 2 million transduced engineered T cells per kilogram of body weight (cells/kg). Therefore, in some aspects, a therapeutically effective dose of transduced engineered T cells is from about 1 million to about 3 million transduced engineered T cells/kg. In certain aspects the therapeutically effective dose is about 2 million transduced engineered T cells/kg. In certain aspects, the therapeutically effective dose is at least about 2 million transduced engineered T cells/kg. In certain aspects, the therapeutically effective dose is at least about 1 million, at least about 2 million, at least about 3 million, at least about 4 million, at least about 5 million, at least about 6 million, at least about 7 million, at least about 8 million, at least about 9 million, at least about 10 million engineered T cells/kg. In other aspects, the therapeutically effective dose may be less than 1 million cells/kg, 1 million cells/kg, 2 million cells/kg, 3 million cells/kg, 4 million cells/kg, 5 million cells/kg, 6 million cells/kg, 7 million cells/kg, 8 million cells/kg, 9 million cells/kg, 10 million cells/kg, more than 10 million cells/kg, more than 20 million cells/kg, more than 30 million cells/kg, more than 40 million cells/kg, more than 50 million cells/kg, more than 60 million cells/kg, more than 70 million cells/kg, more than 80 million cells/kg, more than 90 million cells/kg, or more than 100 million cells/kg. In other aspects, the therapeutically effective dose may be between about 1 million cells to about 2 million cells/kg, about 1 million cells to about 3 million cells/kg, about 1 million cells to about 4 million cells/kg, about 1 million cells to about 5 million cells/kg, about 1 million cells to about 6 million cells/kg, about 1 million cells to about 7 million cells/kg, about 1 million cells to about 8 million cells/kg, about 1 million cells to about 9 million cells/kg, about 1 million cells to about 10 million cells/kg. In some aspects, the total therapeutically effective dose (transduced cells per patient) may be as high as about 1e6 transduced cells, between about 1e6 and about 1e7 transduced cells, between about 1e7 and about 1e8 transduced cells, between about 1e8 and about 1e9 transduced cells, between about 1e9 and about 1e10 transduced cells, between about 1e10 and about 1e11 transduced cells, about 1e11 transduced cells, or over about 1e11 transduced cells. In one aspect, the therapeutically effective dose may be between about 1e8 and about 2e8 transduced cells. One skilled in the clinical and pharmacological arts will be able to determine a therapeutically effective amount through routine experimentation, namely by monitoring a subject's response to administration of a compound and adjusting the dosage accordingly. In certain embodiments, the cell surface receptor that is expressed by the engineered T cells is an anti-CD19 CAR. The anti-CD19 CAR may be a FMC63-28Z CAR or a FMC63-CD828BBZ CAR as set forth in Kochenderfer et al., J Immunother. 2009 September ; 32(7): 689-702, the-which describes methods of constructing the vectors used to produce T cells expressing a FMC63-28Z CAR or a FMC63-CD828BBZ CAR. The therapeutically effective dose of engineered T cells expressing a FMC63-28Z CAR or a FMC63-CD828BBZ CAR may be more than about 1 million to less than about 3 million transduced engineered T cells per kilogram of body weight (cells/kg). The therapeutically effective dose of engineered T cells expressing a FMC63-28Z CAR or a FMC63-CD828BBZ CAR may be more than 1 million to about 2 million transduced engineered T cells per kilogram of body weight (cells/kg). The therapeutically effective dose of engineered T cells expressing a FMC63-28Z CAR or a FMC63-CD828BBZ CAR may be from about 2 million to less than about 3 million transduced engineered T cells/kg. In certain aspects, the therapeutically effective dose of engineered T cells expressing a FMC63-28Z CAR or a FMC63-CD828BBZ CAR is about 2.0 million, about 2.1 million, about 2.2 million, about 2.3 million, about 2.4 million, about 2.5 million, about 2.6 million, about 2.7 million, about 2.8 million, or about 2.9 million transduced engineered T cells/kg. The preferred therapeutically effective dose of engineered T cells expressing a FMC63-28Z CAR or a FMC63-CD828BBZ CAR is about 2 million transduced engineered T cells/kg. In certain aspects, the therapeutically effective dose of engineered T cells expressing a FMC63-28Z CAR or a FMC63-CD828BBZ CAR is at least about 2 million transduced engineered T cells/kg.

A pharmaceutical composition may comprise a population of engineered T cells produced by the methods described herein. The pharmaceutical composition may also include a pharmaceutically acceptable carrier. A pharmaceutically acceptable carrier may be a pharmaceutically acceptable material, composition, or vehicle that is involved in carrying or transporting cells of interest from one tissue, organ, or portion of the body to another tissue, organ, or portion of the body. For example, the carrier may be a liquid or solid filler, diluent, excipient, solvent, or encapsulating material, or some combination thereof. Each component of the carrier must be "pharmaceutically acceptable" in that it must be compatible with the other ingredients of the formulation. It also must be suitable for contact with any tissue, organ, or portion of the body that it may encounter, meaning that it must not carry a risk of toxicity, irritation, allergic response, immunogenicity, or any other complication that excessively outweighs its therapeutic benefits.

The term "about" as used herein means within 5% or 10% of a stated value or a range of values.

### EXAMPLE 1: Preparation of ex vivo gene-modified autologous cells

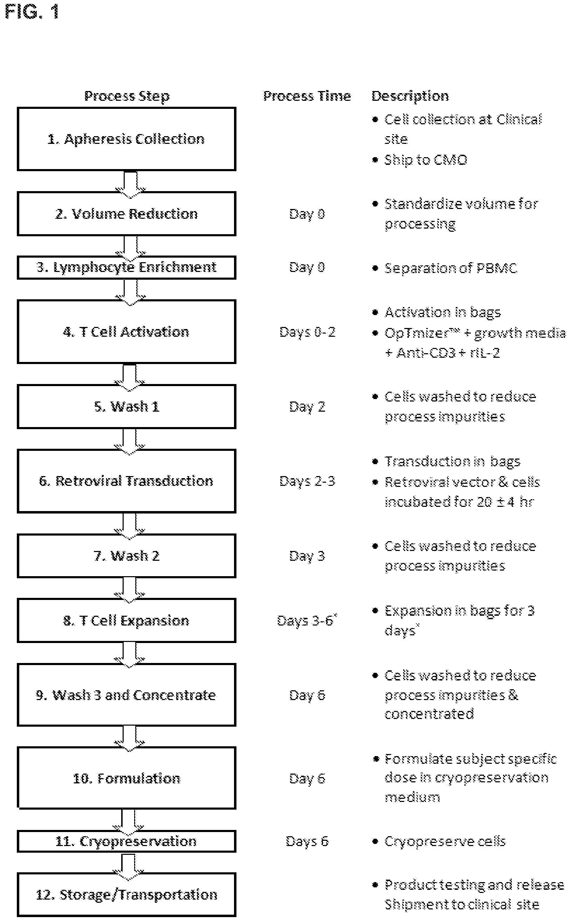

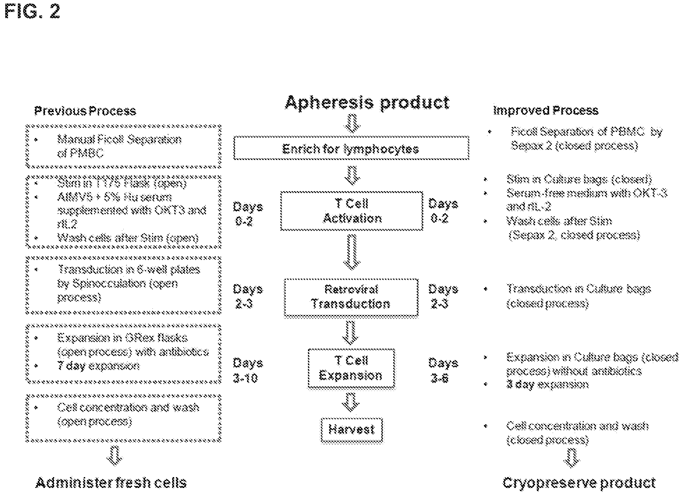

An overview of an exemplary T cell manufacturing process (the "improved" process) is provided in Figure 1. This improved process includes improvements to a traditionally used process of manufacturing T cells (the "previous" process) (see FIG. 2 illustrating these improvements) while maintaining the characteristics of the T cell product. Specifically, the improved process is a closed process that unexpectedly is capable of eliminating the use of serum. Additionally, this improved process uses a single cycle transduction to produce a population of transduced T cells. Further, cells which undergo expansion for a total of 6 days using this process exhibit a more juvenile immuno-phenotypic profile compared with cells that undergo expansion for 10 days cells. This process is capable of reproducibly manufacturing a product with a target number of transfected T cells expressing a chimeric antigen receptor (CAR) to, e.g., CD19; however, these methods apply to T cells transduced with any CAR.

Specifically, the process is designed to be compatible with apheresis product collected using standard apheresis equipment and procedures, enrich the subject's apheresis for lymphocytes and activate the subject's T cells during a defined culture period in the presence of recombinant IL-2 and anti CD3 antibody, provide an ex vivo culture environment where T cells selectively survive and proliferate, transfect the subject's T cells using an engineered retroviral vector to express a CD19 chimeric antigen receptor within a consistent range of transfection efficiency, reduce product related impurities to consistent levels (product related impurities include non-T cell in the starting material from the subject), and reduce process related impurities to consistent levels (process related impurities include growth media, cytokines, and other process reagents).

*Apheresis Collection.* White blood cells were collected (leukapheresis) using standard apheresis equipment, such as Cobe^{®} Spectra, Spectra Optia^{®}, Fenwal^{™} Amicus^{®} or equivalent. The leukapheresis process typically yielded approximately 200-400 mL of apheresis product from patients. The apheresis product may be subjected to the manufacturing process on-site, or optionally shipped at 1-10°C to a facility to undergo the manufacturing process in a different location. Further process steps may be conducted in an ISO 7 cell culture process suite (or similar clean room type environment), as outlined in Figure 1.

*Volume Reduction.* Where appropriate, an improved process volume reduction step was performed using a cell processing instrument such as the Sepax^{®} 2 laboratory instrument (Biosafe SA; Houston, TX) or equivalent, and carried out using a standard aseptic tubing kit. Given the variability in the number of cells and volume of incoming source material from each subject (approximately 200-400 mL), the volume reduction step is designed to standardize the volume of cells to approximately 120 mL. In the event that the apheresis volume is less than 120 mL, the volume reduction step need not be performed, and the cells directly carried to the lymphocyte enrichment step. The volume reduction step is designed to standardize the volume of cells received from each subject, retain mononuclear cells, achieve consistent cell yield and high cell viability, and maintain a closed system to minimize risk of contamination.

*Lymphocyte Enrichment.* Following the Volume Reduction step, the cells were subjected to Ficoll based separation on a cell processing instrument, such as the Sepax^{®} 2 or equivalent, using the separation protocol developed and recommended by the instrument manufacturer (NeatCell Program) and using a standard aseptic tubing kit. The lymphocyte enrichment step reduces product related impurities such as RBCs, and granulocytes, enriches and concentrates the mononuclear cells, washes and reduces process related residuals such as Ficoll, and formulates the cells in growth media in preparation for cell activation, as well as achieving consistent cell yield and high cell viability. The closed system minimizes environmental contamination.

The process may be carried out in an ISO 7 area at ambient temperature and all connections may be conducted either using a sterile tubing welder, or carried out in an ISO 5 laminar flow hood.

*T Cell Activation.* The T Cell Activation step may be carried out either with freshly processed cells from the Lymphocyte Enrichment, or previously cryopreserved cells. In the event that cryopreserved cells are used, the cells may be thawed using developed protocols prior to use.

The T cell Activation step selectively activates T cells to become receptive to retroviral vector transduction, reduces the viable population of all other cell types, achieves consistent cell yield and high T cell viability, and maintains a closed system to minimize the risk of contamination.

*Wash* 1. Following the T Cell Activation step, the cells were washed using cell processing equipment, such as the Sepax^{®} 2 or equivalent, with fresh culture media in a standard aseptic kit using developed protocols by the manufacturer. The cells were optionally concentrated to a final volume of approximately 100 mL in preparation for retroviral vector transduction. The Wash 1 step reduces process related residuals such as anti-CD3 antibody, spent growth media, and cellular debris; achieves consistent cell yield and high T cell viability, maintains a closed system to minimize the risk of contamination; and concentrates and delivers a sufficient number of viable T cells in a small volume appropriate for initiation of transduction.

*Retroviral Transduction.* Activated cells from the Wash 1 step in of fresh cell growth media were transferred to a cell culture bag (Origen Biomedical PL240 or comparable) which has been previously prepared by first coating the bags with a recombinant fibronectin or fragments thereof such as RetroNectin^{®} (Takara Bio, Japan), and subsequently incubated with retroviral vector according to defined procedures prior to introduction of the activated cells. RetroNectin^{®} coating (10 µg/mL) was carried out at a temperature of 2-8°C for 20± 4 hr, washed with dilute buffer, and subsequently incubated with thawed retroviral vector for approximately 180 - 210 min at 37± 1 °C and 5 ± 0.5% CO₂. After the addition of cells to the bag, the transduction was carried out for 20 ± 4 hr at 37 ± 1 °C and 5 ± 0.5% CO₂. The retroviral transduction step cultures the activated T cells in the presence of the retroviral vector under controlled conditions in order to allow for efficient transduction to take place, achieves consistent cell yield and high cell viability, and maintains a closed system in order to minimize the risk of contamination.

*Wash* 2. Following the retroviral transduction step, the cells were washed with fresh growth media using cell processing equipment, such as the Sepax^{®} 2 or equivalent, in a standard aseptic kit using protocols developed by the manufacturer, and the cells were concentrated to a final volume of approximately 100 mL in preparation for the expansion step. The Wash 2 step is designed to reduce process related residuals such as retroviral vector particles, vector production process residuals, spent growth media, and cellular debris achieve consistent cell yield and high cell viability; maintain a closed system to minimize the risk of contamination; and exchange spent growth media for fresh media with a target number of cells in a specified volume appropriate for initiation of expansion step.

*T Cell Expansion.* Cells from the Wash 2 step were aseptically transferred to a culture bag (Origen Biomedical PL325 or equivalent) and diluted with fresh cell growth media and cultured for approximately 72 hr at 37 ± 1 °C and 5 ± 0.5% CO₂. The cell density was measured daily starting on Day 5. Because doubling times of the T cells may vary slightly from subject to subject, additional growth time beyond 72 hr (i.e., 3-6 days) may be necessary in the event that the total cell number is insufficient to deliver a target dose of CAR-positive T cells/kg of subject weight. The T cell expansion step is designed to culture the cells under controlled conditions in order to produce a sufficient number of transduced cells for delivering an efficacious dose, maintain a closed system to minimize risk of contamination, and achieve consistent cell yield and high cell viability. One such efficacious dose or target dose includes 2 × 10⁶ FMC63-28Z CAR positive or FMC63-CD828BBZ CAR positive T cells/kg (± 20%) of subject weight that were produced via transduction with either the MSGV-FMC63-28Z retroviral vector or the MSGV-FMC63-CD828BBZ retroviral vector, respectively, both of which are described in detail in Kochenderfer et al., J Immunother. 2009 September; 32(7): 689-702

*Wash 3 and Concentrate.* Following the T cell expansion step, the cells were washed with 0.9% saline using a cell processing instrument, such as the Sepax^{®} 2 or equivalent, in a standard aseptic kit using developed protocols by the manufacturer, and the cells were concentrated to a final volume of approximately 35 mL in preparation for the formulation and cryopreservation. The wash 3 step is designed to reduce process related residuals such as retroviral production process residuals, spent growth media, and cellular debris; achieve consistent cell yield and high cell viability; and maintain a closed system to minimize risk of contamination.

Once the cells have been concentrated and washed into 0.9% saline, an appropriate cell dose may be formulated for preparation of the final cryopreserved product. The cells were prepared for cryopreservation and cryopreserved according to the methods provided below in Example 4.

### EXAMPLE 2: Growth performance of T cells expanded in cell culture bags

The embodiments described herein provide for efficient production of an engineered autologous T cell therapy in 6 days. The following improvements over the art were achieved: a shortened process to 6 days instead of either 24, 14, or 10 days as previously used (this reduces the number of tests needed for product release (including RCR testing)); improved T cell products, including a higher proportion of juvenile T cells for increased potency and effectiveness; a culture which can be initiated with a larger number of cells to offset the shorter manufacturing time; a closed system in which to perform the steps of the methods described herein; identification of human serum-free culture conditions that support T cell growth; single cycle retrovirus transduction in bags; cell culture activation and expansion performed in bags rather than flasks; and provision of a frozen product. These improvements were used for the development and commercialization of novel engineered peripheral blood autologous T cell therapeutics (eACT) for the treatment of multiple cancer indications. T cells resulting from the development program maintained the same phenotypic and activity profile as cells propagated by previous methods.

*Generation of engineered T cells.* Figure 2 shows an overview of the T cell manufacturing process with the improvements described herein in the improved process. Briefly, peripheral blood mononuclear cells (PBMCs) were obtained from subjects having B cell malignancies by apheresis, split, and processed side-by-side using prior techniques, and the improved techniques described herein. Five studies evaluated all process steps through growth at Day 6, but not the final wash and cryopreservation operations. In two additional studies, the improved process was executed from the initial processing of apheresis material through the final formulation and freeze steps, again using apheresis product from lymphoma patients.

The apheresis product (or "sample") was enriched for lymphocytes by Ficoll Separation of the PBMCs by a closed Sepax 2 process. The lymphocytes were then grown in closed culture bags in serum-free medium (OpTmizer^{™}, Life Technologies) supplemented with a developmental prototype supplement (T cell SR Media Supplement, Life Technologies) and stimulated for T cell activation with anti-CD3 antibody and rIL-2 (recombinant IL-2) for 48 hours (days 0-2). The activated T cells were then washed using a closed Sepax 2 process.

On days 2-3 of the manufacturing process, the activated T cells were transduced with an anti-CD19 CAR using a gamma retroviral vector. The transduction was accomplished in a closed system as follows. A closed cell culture bag (i.e., Origen PermaLife^{™} PL240 bag) was coated with RetroNectin^{®} at 2-10 µg/mL, then the RetroNectin^{®} was removed and the bag was washed with buffered saline. The gamma retrovirus was then introduced in the closed system bag, followed by an incubation period. The activated T cells were then added directly into the bag containing the retroviral vector followed by an overnight incubation at 37 °C. The material from the RetroNectin^{®}-coated culture bag was removed and placed in a separate cell culture bag for cell expansion. An optional wash step may be added prior to cell expansion. The transduced T cells were expanded in a closed bag system without antibiotics for 3 days (days 3-6). The resulting engineered T cells were then harvested and cryopreserved (cryopreservation is an optional step).

*Engineered T cell phenotype.* The engineered T cells were analyzed by fluorescence-activated cell sorting (FACS) to (i) confirm CAR gene expression, (ii) confirm T cell population purity, and (iii) determine cell phenotypes present in the population of engineered T cells using cell surface expression of T cell subset markers CCR7, CD45RA, CD62L and functional competence markers CD27 and CD28 .

*Engineered T cell activity.* The engineered T cells were also analyzed using an *in vitro* co-culture bioassay to measure the production of interferon gamma (IFNγ) by the engineered T cells after co-culture with antigen (Ag) positive (i.e., CD19+) target cells. The engineered T cells were also analyzed for intracellular production of interferon gamma (IFNγ) by the engineered T cells and expression of CD107a after co-culture with Ag positive target cells by FACS.

*Growth studies.* T cell growth and viability were evaluated in each experiment to assure that cell growth was robust, consistent and similar (or better) than that in conventional studies.

*Cryopreservation.* Cells were washed on day 6 with normal saline, then HSA was added to 5%, and the cells were mixed 1:1 with CryoStor^{™} 10 (BioLife Solutions^{™}). Cells were then frozen in a controlled rate freezer using a defined freeze cycle, then stored in vapor phase liquid nitrogen. It was shown that cells between about 1 × 10⁶ and 1.5 × 10e7per mL can be cryopreserved and thawed successfully by this process. Saline with HSA was as good or better than other solutions tested (e.g., PL/D5), and HSA improved freeze-thaw recovery.

Cells were evaluated for viability at thaw by trypan blue exclusion as well as FACS (FACS based staining for Annexin V as well as 7AAD). Cells retained their phenotype and biological function as measured by IFN-gamma after thaw.

### Results

Growth studies were conducted in serum-free media in conjunction with media growth supplements. In these studies, performance was variable, and success was defined as a medium that resulted in a phenotype similar to that of medium (AIMV) containing 5% human serum. The serum free medium used in the methods described above resulted in excellent T cell growth and a phenotype that was similar to growth in AIMV. One unexpected observation was that to achieve excellent growth and viability, the cells needed to be passed when the cell density reached approximately 1.5 × 10⁶/mL. If cells were not passed at about this cell concentration, viability could drop. In the range of approximately 0.4 to 1.5e6/mL, however, cells grew well with a doubling time of 24 hours or less in culture at 37 °C in either flasks or closed bag systems.

The process to generate engineered T cells where a new receptor gene is introduced into T cells using a gamma retroviral vector requires that cells be in active growth so that they can be successfully transduced. Here, the T cells were transduced with an anti-CD19 CAR, but the process described herein may be used for any CAR or TCR. It was demonstrated that human T cell growth can be stimulated in the OpTmizer^{™} medium using anti-CD3 antibody and IL2 either in open T flasks or in a closed cell culture bag system. FACS was used to demonstrate that cells stained with CFSE grew equally well in OpTmizer^{™} medium or AIMV plus 5% human serum during this stimulation. Although the T cell growth was seen at other incubation times, it was demonstrated that a 2-day incubation with anti-CD3 antibody and IL2 is optimal to get the cells actively growing in OpTmizer^{™} medium.

A variety of conditions were evaluated to look at transduction in OpTmizer^{™} medium in a closed bag system. One novel aspect of this invention is the specific order of the steps that were developed to achieve transduction in a closed bag system in OpTmizer^{™} medium. The process described has the advantage that it is operationally simple, yet provides a transduction frequency that is similar to previously used conditions. It was learned that steps previously included in transduction protocols were not necessary (for example blocking coated surfaces with proteins such as HSA). Transduction frequency is not impacted by a wash of the cells after removal from the RetroNectin^{®}-coated cell culture bag.

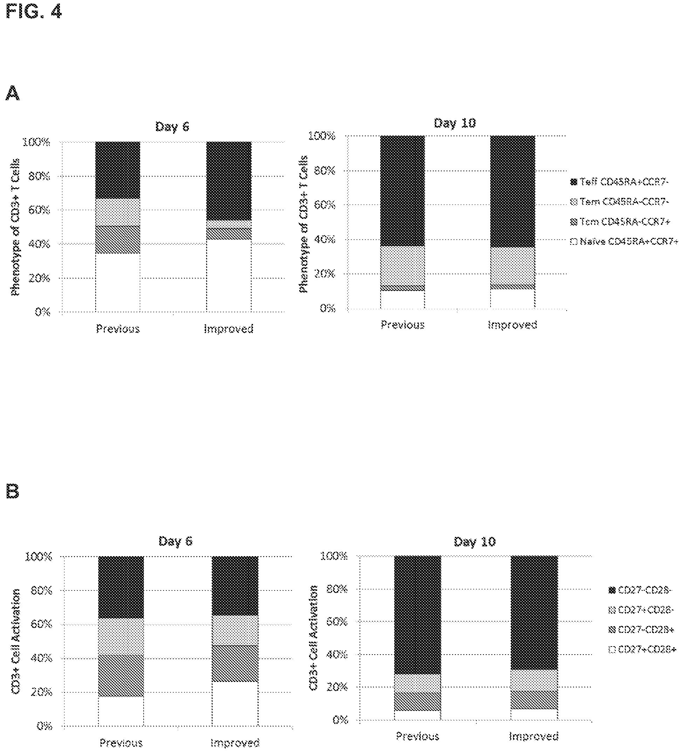

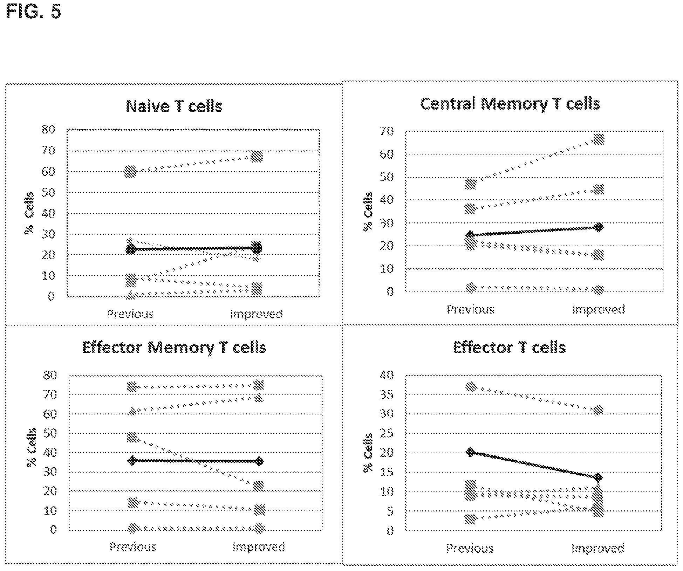

Phenotypic analysis of cells at either 6 days or 10 days post initiation of the stimulation process revealed that in OpTmizer^{™} medium the cells are generally similar to cells grown in AIMV medium in similar bags or in the plate system in general use (see FIGS. 4-5). Similarly, the cells produced in the simple, closed process bag system in OpTmizer^{™} medium are capable of producing IFNgamma in response to Ag-positive target cells in an *in vitro* co-culture assay, which demonstrates that the T cells manufactured by this enhanced process are biologically active.

Table 1 below shows IFNgamma production (pg/ml) on day 6 using the improved process described herein.

**Table 1**

| | **CD19+ Cell Lines** | | | | **CD19- Cell Lines** | |
|---|---|---|---|---|---|---|
| | **Toledo** | **Nalm6** | **CD19-K562** | | **NGFR-K562** | **CEM** |
| **UT previous** | 0 | 5 | 17 | | 17 | 5 |
| **UT improved** | 0 | 228 | 56 | | 5 | 17 |
| **TD previous** | 11269 | 13986 | 64911 | | 43 | 17 |
| **TD improved** | 11101 | 16883 | 104324 | | 70 | 0 |
| **Control** | 11942 | 19818 | 81165 | | 30 | 5 |

| | | | | | | |
|---|---|---|---|---|---|---|
| Samples were from a full scale engineering run UT - untransduced; TD - transduced | | | | | | |

Another unexpected observation was that by simply diminishing the duration of culture from 10 days or longer to the present 6 days resulted in a more juvenile T cell distribution with increased representation of naive, central memory cells and decreased representation of differentiated effector T cells (See FIGS. 4-5). This has a beneficial impact on product potency and other attributes. Specifically, sufficient cells are able to be collected from the expanded product after only 3 days in culture. The total time from initiation of stimulation to harvest of expanded, transduced cells is 6 days, which supports a dose of approximately 1-2 × 10⁸ CAR-positive cells (FIG. 6). If a larger number of cells are required, the cells continue to grow robustly in bags and a cell culture at 10 days or longer can be harvested. Alternatively, a higher number of cells in the starting population can be utilized to generate a larger cell population in the 6 day period.

Additionally, two studies were conducted at full scale with apheresis from lymphoma patients wherein the Day 6 cells were further washed in 0.9% saline, formulated in the final product formulation and cryopreserved. The Day 6 cell product was evaluated pre- and post-thaw for a number of parameters. There was no significant difference in the percentage of CAR-positive T cells 3 days post-thaw relative to the pre freeze level, suggesting that the cryopreservation protocol is not detrimental to CAR expression. In addition, the CAR-positive cells continued to show CD19-specific antigen recognition as measured by IFN-gamma release following co-culture with CD19-positive targets. The viability of the cells at thaw was 90% and 79%, respectively, for the two products tested.

### EXAMPLE 3: Development of transduction conditions in a closed system

Previously, transduction of PBMCs was carried out in non-tissue culture treated 6 well plates. Plates were coated with RetroNectin^{®} at 10 µg/mL overnight at 2-8°C, or for 2 hrs at room temperature. After incubation, RetroNectin^{®} was removed, and plates were blocked with 2.5% HSA for 30 min, followed by a wash with HBSS + 5 mM HEPES. In the plate-based process, retroviral vector was applied into the coated well and spun in a centrifuge, followed by removal of approximately 75% of the viral supernatant, followed by addition of the cells for transduction by spinnoculation.

As provided herein, three studies were completed to optimize the concentration of RetroNectin^{®} for transduction of PBMCs in closed cell culture bags, and to determine if the HSA wash and viral supernatant removal impacted transduction. The first experiment was carried out in Origen PermaLife^{™} PL07 bags, where cell activation, transduction, and expansion were conducted in AIM V^{®} medium + 5% human serum. A RetroNectin^{®} concentration range from 2-40 µg/mL was evaluated in PBMCs from three separate donors. There were no significant differences between transduction in plates vs. transduction in bags at 10 and 40 µg/mL RetroNectin^{®} concentration, or transduction carried out without HSA blocking at the 95% confidence level. However, at the same confidence level, reduction of RetroNectin^{®} concentration to 2 µg/mL, or removal of retroviral vector from the bag prior to transduction, appeared to reduce the transduction efficiency moderately as displayed in Table 2 below.

**Table 2. Impact of RetroNectin^{®} concentration, HSA blocking, and retroviral vector removal on transduction in cell culture bags compared with transduction in plates.**

| | **% CAR+ CD3+** | | | | | | |
|---|---|---|---|---|---|---|---|
| | **Untransduced** | **TD² in Plate** | **TD in Bag (2 µg/mL)** | **TD in Bag (10 µg/mL)** | **TD in Bag (40 µg/mL)** | **TD³ in Bag, no HSA Block** | **TD³ in bag Vector remove** |
| PT1 ¹ | 0.670% | 82.12% | 74.80% | 81.63% | 76.62% | 77.26% | 67.70% |
| PT2 ¹ | 0.19% | 80.25% | 73.24% | 78.39% | 79.63% | 82.75% | 59.55% |
| PT3 ¹ | 0.81% | 82.78% | 72.39% | 79.10% | - | 75.80% | 65.36% |
| Mea n | 0.57% | 81.70% | 73.47% | 79.70% | 77.35% | 75.13% | 63.62% |
| Std. Dev | 0.33% | 1.31% | 1.22% | 1.71% | 2.01% | 8.89% | 5.76% |
| ¹PT1, PT2 and PT3 refer to PBMCs from three separate donors. | | | | | | | |
| ²TD refers to transduction | | | | | | | |
| ³Conditions without an HSA block or with retroviral vector removal were conducted at a RetroNectin^{®} concentration of 10 µg/mL. | | | | | | | |

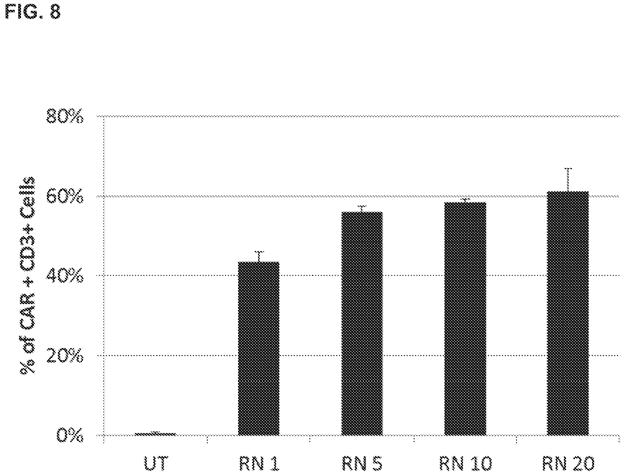

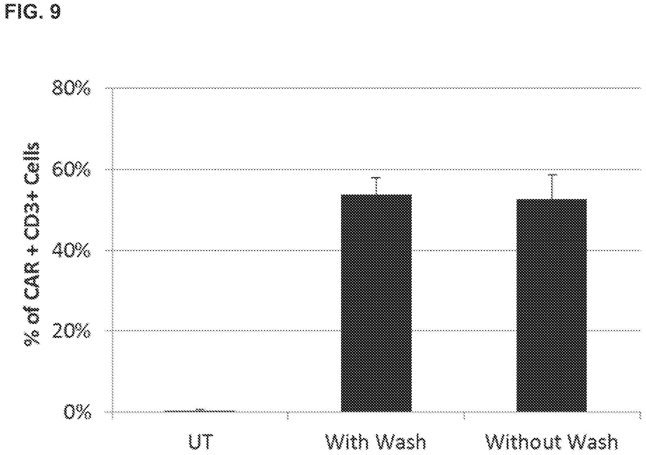

A second study in AIM V^{®} medium + 5% human serum in Origen PermaLife^{™} PL07 bags using PBMCs from two separate donors confirmed the results from the first study and demonstrated that maximum transduction efficiency in bags occurs in the range of 1-20 µg/mL RetroNectin^{®} (see FIG. 8). Additionally, an HSA block step does not enhance the process or increase transduction efficiency (see FIG. 9). Further, the study showed that transduced cell phenotype (CD45RA/CCR7) is not impacted by elimination of the HSA block step.

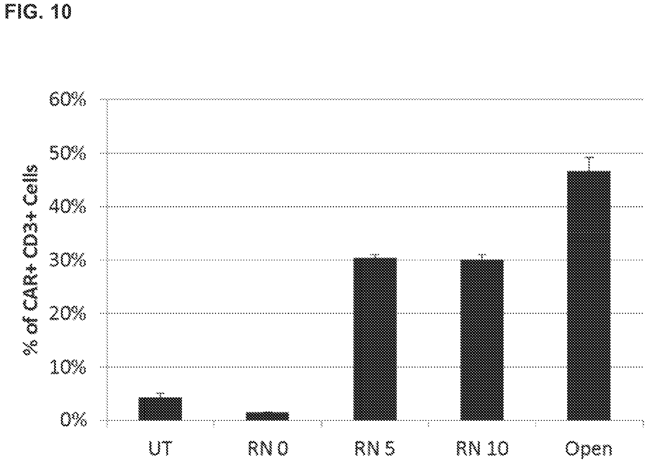

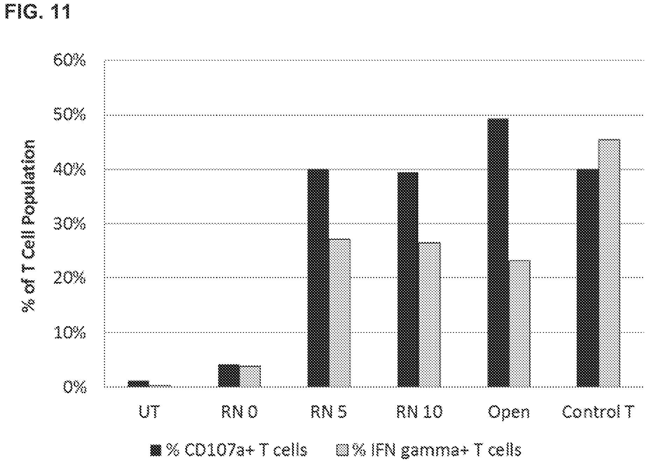

In a third study, PBMCs from 2 donors were stimulated in either OpTmizer^{™} + 2.5% supplement in Origen PermaLife^{™} PL70 bag or AIM V^{®} + 5% HSA for 2 days. On day 2, cells were washed and transduced with the retroviral vector in PL30 or 6-well plates. Cell concentration during transduction was 0.5 × 10⁶/mL. On day 3, transduced cells were either transferred into T175 flasks (as control) or PL30 bags. On Day 6 and 7, cells were evaluated for potency in the co-culture assay and by FACS for CAR expression and phenotype. Figure 10 shows the impact of RetroNectin^{®} concentration on transduction frequency where RetroNectin^{®} above 5 µg/mL had no impact on transduction frequency in bags. Figure 11 shows that the activity of cells tested in these conditions was similar when measures of cellular activation (CD107a expression and IFN-gamma production) in response to CD19 antigen recognition on target cells were evaluated. In this case, transduced cells were incubated with CD19-positive Nalm6 cells for four hours, followed by staining for cell surface expression of CD107a, and for intracellular IFN-gamma production.

Thus, in accordance with the methods provide herein, a process is supported in which bags are: coated with RetroNectin^{®} at 10 µg/mL; transduction is executed in bags using OpTmizer^{™} medium + 2.5% supplement; a blocking step with HSA had no impact on transduction efficiency or impact on cell potency or phenotype; transduction in bags yielded T cell products with similar phenotype to transduction in plates; and retroviral vector removal prior to addition of cells to the bag during transduction did not increase, and may slightly decrease, overall transduction frequency.

### EXAMPLE 4: Development of cryopreservation step for manufactured anti-CD19 CAR+ T cells

A series of development studies were conducted to determine the optimal conditions for cryopreservation of manufactured anti-CD19 CAR T cells. Studies were designed to establish conditions for high viability at thaw, a frozen product formulation, an optimized freeze protocol, and to determine the impact of freeze thaw on cell phenotype and potency. The following analytical measures were employed to assess performance: cell count by trypan blue exclusion pre- and post-thaw, annexin staining by FACS post thaw, FACS staining to determine CAR+ T cells and phenotype (CCR7, CD45RA), growth in culture of cryopreserved cells after thaw, and potency by IFN-gamma production after co-cultivation with CD19+ cells.

PBMCs transduced with retroviral vector were used to assess the above-mentioned performance measures. In development studies, cryopreserved cells in the concentration range of 3 - 12 × 10⁶/mL were used in a final cryopreserved volume of 20 mL. The cell density of actual clinical products is expected to fall within this range, based on subject body weight and CAR transduction frequency. Studies were conducted in either OriGen CS50 bags or AFC KryoSure^{®} 20-F bags with no noticeable differences.

Transduced cells were washed and re-suspended in a solution containing either 0.9% saline or a 1:1 mixture of PLASMA-LYTE^{®} A and D5 half normal saline (5% dextrose / 0.45% NaCl), with or without human serum albumen (HSA). Cells were then mixed at a ratio of 1:1 or 1:2 with CryoStor^{®} CS10. In the various studies, cells were cryopreserved in a controlled rate freezer (CRF), stored in vapor phase LN2 for > 2 days, then thawed and evaluated for viability, CAR expression, phenotype and activity. In some experiments, cells were mixed 1:1 with 80% human AB serum + 20% DMSO as a control.

Cell recovery at thaw was enhanced when 2.5% final concentration of HSA was included in the cryopreserved product (Table 3). In addition, cells frozen with HSA maintained a higher viability, began growing more rapidly when placed back into culture and regained a high cell viability more quickly (Table 4).

**Table 3. Cell recovery immediately post-thaw of cryopreserved anti CD19 CAR+ T cells at different dilutions of cryopreservatives.**

| **Formulation** | **Total cells frozen (× 10⁶)** | | **Total cells recovered (× 10⁶)** | | **% Recovery post-thaw** | |
|---|---|---|---|---|---|---|
| | **No HSA** | **HSA** | **NoHSA** | **HSA** | **No HSA** | **HSA** |
| CS10 only | 68 | 74 | 52 | 58 | 77% | 78% |
| 2:1 CS10:Diluent | 68 | 74 | 51 | 63 | 76% | 85% |
| 1:1 CS10:Diluent | 68 | 74 | 45 | 69 | 66% | 93% |
| 1:1 Serum*:Diluent | 68 | 74 | 46 | 67 | 67% | 91% |
| Diluent refers to a cell suspension in a 1:1 mixture of PLASMA-LYTE^{®} A and D5 half normal saline (5% dextrose / 0.45% NaCl) | | | | | | |
| *Serum - 80% human AB serum/20% DMSO | | | | | | |

**Table 4. Cell recovery and growth post thaw of cryopreserved anti CD19 CAR+ T cells in various dilutions of cryopreservatives.**

| **Sample ID** | **HSA** | **Viability** | | | | **Cell count (× 10⁶)** | | | |
|---|---|---|---|---|---|---|---|---|---|
| | | **Day 0** | **Day 1** | **Day 2** | **Day 3** | **Day 0¹** | **Day 1** | **Day 2** | **Day 3** |
| CS10 only | No | 96% | 81% | 86% | 90% | 20 | 19 | 33 | 58 |
| 2:1 CS10:Diluent | No | 96% | 81% | 86% | 89% | 20 | 14 | 29 | 49 |
| 1:1 CS10:Diluent | No | 95% | 83% | 86% | 91% | 20 | 21 | 36 | 66 |
| 1:1 Serum² :Diluent | No | 93% | 86% | 92% | 97% | 20 | 10 | 32 | 64 |
| CS10 only | Yes | 95% | 90% | 92% | 93% | 20 | 26 | 44 | 82 |
| 2:1 CS10:Diluent | Yes | 97% | 88% | 93% | 95% | 20 | 23 | 41 | 82 |
| 1:1 CS10:Diluent | Yes | 96% | 86% | 91% | 93% | 20 | 20 | 37 | 68 |
| 1:1 Serum² :Diluent | Yes | 95% | 87% | 94% | 96% | 20 | 20 | 36 | 66 |
| Diluent refers to a cell suspension in a 1:1 mixture of PLASMA-LYTE^{®} A and D5 half normal saline (5% dextrose / 0.45% NaCl) | | | | | | | | | |
| ¹Total number of cells placed into culture on Day 0 | | | | | | | | | |
| ² Serum - 80% human AB serum/20% DMSO | | | | | | | | | |

Studies comparing PLASMA-LYTE^{®} A/D5 half normal saline to 0.9% saline showed that there was no significant difference in recovery at thaw, cell growth performance in culture after thaw or phenotype (Table 5). Also, there was no improvement in these parameters when the cells were diluted 1:2 with CryoStor^{®} CS10 vs. 1:1 with CryoStor^{®} CS10. In most experiments with 5% human serum in the T cell diluents with a final concentration of HSA in the cryopreserved product of 2.5%, performance was equal to or exceeded that of cells frozen in 1:1 with 80% human AB serum/20% DMSO. A decision was made therefore to select a final cryopreserved product where cells are washed with 0.9% normal saline, HSA is added to 5%, then cells are diluted 1:1 with CryoStor^{®} CS10.

**Table 5. Performance of cells diluted in 0.9% normal saline vs. PLASMA-LYTE^{®}/D5 half normal saline.**

| **Sample** | **Viability (%)** | | | | **Cell count (× 10⁶ cells)** | | | | |
|---|---|---|---|---|---|---|---|---|---|
| | **Day 0** | **Day 1** | **Day 2** | **Day 3** | **# of cells recovered at thaw¹** | **# of cells placed into culture** | **Day 1** | **Day 2** | **Day 3** |
| 2:1 NS | 91 | 86 | 91 | 85 | 163 | 20 | 21 | 22 | 29 |
| 2:1 NS | 90 | 84 | 85 | 86 | 155 | 20 | 17 | 21 | 26 |
| 1:1 NS | 91 | 81 | 88 | 85 | 175 | 20 | 15 | 22 | 42 |
| 1:1 NS | 93 | 86 | 84 | 88 | 163 | 20 | 20 | 21 | 45 |
| 2:1 PL/D5 | 91 | 87 | 87 | 84 | 161 | 20 | 16 | 24 | 23 |
| 2:1 PL/D5 | 91 | 83 | 85 | 88 | 172 | 20 | 20 | 23 | 27 |
| 1:1 PL/D5 | 95 | 87 | 89 | 87 | 191 | 20 | 18 | 25 | 24 |
| 1:1 PL/D5 | 93 | 87 | 88 | 86 | 203 | 20 | 17 | 24 | 21 |
| ¹200 × 10⁶ cells were frozen in each bag in a total volume of 20 mL PL/D5 - 1:1mixture of PLASMA-LYTE^{®} A and D5 half normal saline (5% dextrose / 0.45% NaCl); NS - Normal Saline | | | | | | | | | |

Freezing cycle development was carried out on the selected formulation of 0.45% normal saline, 2.5% HSA and 50% CryoStor^{®} CS10, and a final product volume of approximately 50-60 mL in Origen^{™} CS250 bags. All runs were conducted individually, using a single medium formulation and volume for each run. Air was purged from the bag and product temperature was monitored by attaching a thermocouple to the external surface of the bag. Individual bags were placed into a freezing cassette designed to accommodate the CS250 bag, such as Custom BioGenic Systems^{™}, Part Number ZC021, and placed in the middle shelf of a freezing rack in the controlled-rate freezer.

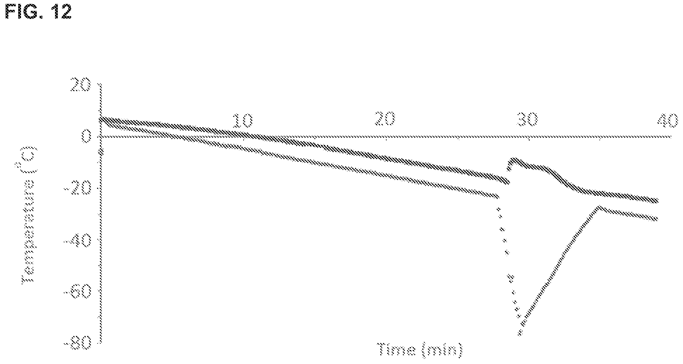

A series of freeze cycles were evaluated to determine the point at which the 0.45% normal saline, 2.5% HSA and 50% CryoStor^{®} CS10 spontaneously nucleated to assist in deciding where to initiate the temperature spike. After each run, modifications to the freezing protocol were made until a satisfactory sample temperature profile was produced. Criteria for the creation of a satisfactory sample were that the cold spike offset the heat of fusion to the greatest possible degree and that the sample adhered to a 1°C/minute cooling rate. The optimized protocol for the 50 mL bag in the Controlled Rate Freezer (CRF) is displayed in Table 6, and the corresponding temperature profile of the chamber and product are displayed in Figure 12.

**Table 6. Optimized freeze cycle for the anti CD19 CAR+ T cells product formulation in the controlled rate freezer.**

| **Step** | **Action** | **Target Chamber Temperature** |
|---|---|---|
| 1 | Wait at 4 °C | NA |
| 2 | Ramp 1.0 °C/mi | -23.0 °C |
| 3 | Ramp 30.0 °C/min | -75.0 °C |
| 4 | Ramp 10.0 °C/min | -28.0 °C |
| 5 | Ramp 1.0 °C/min | -40.0 °C |
| 6 | Ramp 10.0 °C/min | -90.0 °C |

## Claims

1. A method for manufacturing T cells
which express a cell surface receptor that recognizes a specific antigenic moiety on the surface of a target cell, the method comprising
(a) enriching a population of lymphocytes obtained from a donor subject;
(b) stimulating the population of lymphocytes with one or more T-cell stimulating agents to produce a population of activated T cells, wherein the stimulation is performed in a closed system using serum-free culture medium;
(c) transducing the population of activated T cells with a viral vector comprising a nucleic acid molecule which encodes the cell surface receptor, using a single cycle transduction to produce a population of transduced T cells, wherein the transduction is performed in a closed bag system using serum-free culture medium, wherein the bag is coated with a recombinant human fibronectin protein or fragment thereof; and
(d) expanding the population of transduced T cells for a predetermined time to produce a population of engineered T cells, wherein the expansion is performed in a closed system using serum-free culture medium.

2. The method of claim 1, wherein the cell surface receptor is a T cell receptor (TCR) or a chimeric antigen receptor (CAR).

3. The method of claim 1, wherein the target cell is a cancer cell.

4. The method of claim 3, wherein the cancer cell is a B cell malignancy.

5. The method of claim 3, wherein the cell surface receptor is an anti-CD19 CAR.

6. The method of claim 1, wherein the one or more T-cell stimulating agents are an anti-CD3 antibody and IL-2.

7. The method of claim 1, wherein the viral vector is a retroviral vector.

8. The method of claim 7, wherein the retroviral vector is an MSGV1 gamma retroviral vector.

9. The method of claim 1, wherein the predetermined time for expanding the population of transduced T cells is 3 days.

10. The method of claim 1, wherein the time from enriching the population of lymphocytes to producing the engineered T cells is 6 days.

11. The method of claim 10, wherein the engineered T cells are for use in the treatment of a cancer patient, wherein the cancer patient and the donor subject are preferably the same individual.

12. The method of claim 1, wherein the closed system is a closed bag system.

13. The method of claim 1 or 12, wherein the bag is not blocked with HSA during the transduction step.

14. The method of claim 1, wherein the population of cells comprises naive T cells, wherein preferably about 35-43%, at least about 35%, or at least about 43% of the population of engineered T cells comprise naive T cells.

## Patentansprüche

1. Verfahren zur Herstellung von T-Zellen,
die einen Zelloberflächenrezeptor exprimieren, der eine spezifische antigene Einheit auf der Oberfläche einer Zielzelle erkennt, das Verfahren umfassend
(a) Anreichern einer Population von Lymphozyten, die von einem Donorsubjekt stammen;
(b) Stimulieren der Lymphozytenpopulation mit einem oder mehreren T-Zell-stimulierenden Mitteln, um eine Population von aktivierten T-Zellen zu erzeugen, wobei die Stimulierung in einem geschlossenen System unter Verwendung eines serumfreien Kulturmediums durchgeführt wird;
(c) Transduzieren der Population aktivierter T-Zellen mit einem viralen Vektor, umfassend ein Nukleinsäuremolekül, das den Zelloberflächenrezeptor umfasst, unter Verwendung einer Einzelzyklustransduktion, um eine Population transduzierter T-Zellen zu erzeugen, wobei die Transduktion in einem geschlossenen Beutelsystem unter Verwendung eines serumfreien Kulturmediums durchgeführt wird, wobei der Beutel mit einem rekombinanten menschlichen Fibronektinprotein oder einem Fragment davon beschichtet ist; und
(d) Expandieren der Population der transduzierten T-Zellen für eine vorbestimmte Zeit, um eine Population von manipulierten T-Zellen zu erzeugen, wobei die Expansion in einem geschlossenen System unter Verwendung eines serumfreien Kulturmediums durchgeführt wird.

2. Verfahren nach Anspruch 1, wobei der Zelloberflächenrezeptor ein T-Zellrezeptor (TCR) oder ein chimärer Antigenrezeptor (CAR) ist.

3. Verfahren nach Anspruch 1, wobei die Zielzelle eine Krebszelle ist.

4. Verfahren nach Anspruch 3, wobei es sich bei der Krebszelle um ein B-Zell-Malignom handelt.

5. Verfahren nach Anspruch 3, wobei der Zelloberflächenrezeptor ein Anti-CD19 CAR ist.

6. Verfahren nach Anspruch 1, wobei es sich bei dem einen oder den mehreren T-Zell-stimulierenden Mitteln um einen Anti-CD3-Antikörper und IL-2 handelt.

7. Verfahren nach Anspruch 1, wobei der virale Vektor ein retroviraler Vektor ist.

8. Verfahren nach Anspruch 7, wobei der retrovirale Vektor ein MSGV1 gamma retroviraler Vektor ist.

9. Verfahren nach Anspruch 1, wobei die vorbestimmte Zeit für die Expansion der Population der transduzierten T-Zellen 3 Tage beträgt.

10. Verfahren nach Anspruch 1, wobei die Zeit vom Anreichern der Lymphozytenpopulation bis zum Herstellen der manipulierten T-Zellen 6 Tage beträgt.

11. Verfahren nach Anspruch 10, wobei die gentechnisch veränderten T-Zellen zur Verwendung bei der Behandlung eines Krebspatienten bestimmt sind, wobei der Krebspatient und das Donorsubjekt vorzugsweise dasselbe Individuum sind.

12. Verfahren nach Anspruch 1, wobei das geschlossene System ein geschlossenes Beutelsystem ist.

13. Verfahren nach Anspruch 1 oder 12, wobei der Beutel während des Transduktionsschritts nicht mit HSA blockiert wird.

14. Verfahren nach Anspruch 1, wobei die Zellpopulation naive T-Zellen umfasst, wobei vorzugsweise etwa 35-43 %, mindestens etwa 35 % oder mindestens etwa 43 % der Population der manipulierten T-Zellen naive T-Zellen umfassen.

## Revendications

1. Procédé permettant de fabriquer des cellules T
qui expriment un récepteur de surface cellulaire qui reconnaît un fragment antigénique spécifique sur la surface d'une cellule cible, le procédé comprenant
(a) l'enrichissement d'une population de lymphocytes obtenus auprès d'un sujet donneur ;
(b) la stimulation de la population de lymphocytes avec un ou plusieurs agents de stimulation de cellules T pour produire une population de cellules T activées, dans lequel la stimulation est mise en oeuvre dans un système fermé à l'aide de milieu de culture exempt de sérum ;
(c) la transduction de la population de cellules T activées avec un vecteur viral comprenant une molécule d'acide nucléique qui code pour le récepteur de surface cellulaire, à l'aide d'une transduction à cycle unique pour produire une population de cellules T transduites, dans lequel la transduction est mise en oeuvre dans un système de sac fermé à l'aide de milieu de culture exempt de sérum, dans lequel le sac est revêtu d'une protéine de fibronectine humaine recombinée ou d'un fragment de celle-ci ; et
(d) l'expansion de la population de cellules T transduites pendant un temps prédéterminé pour produire une population de cellules T modifiées artificiellement, dans lequel l'expansion est mise en oeuvre dans un système fermé à l'aide de milieu de culture exempt de sérum.

2. Procédé selon la revendication 1, dans lequel le récepteur de surface cellulaire est un récepteur de cellules T (TCR) ou un récepteur antigénique chimérique (CAR).

3. Procédé selon la revendication 1, dans lequel la cellule cible est une cellule cancéreuse.

4. Procédé selon la revendication 3, dans lequel la cellule cancéreuse est une malignité à cellules B.

5. Procédé selon la revendication 3, dans lequel le récepteur de surface cellulaire est un CAR anti-CD19.

6. Procédé selon la revendication 1, dans lequel le ou les agents de stimulation de cellules T sont un anticorps anti-CD3 et IL-2.

7. Procédé selon la revendication 1, dans lequel le vecteur viral est un vecteur rétroviral.

8. Procédé selon la revendication 7, dans lequel le vecteur rétroviral est un vecteur rétroviral MSGV1 gamma.

9. Procédé selon la revendication 1, dans lequel le temps prédéterminé pour l'expansion de la population de cellules T transduites est de 3 jours.

10. Procédé selon la revendication 1, dans lequel le temps à partir de l'enrichissement de la population de lymphocytes jusqu'à la production des cellules T modifiées artificiellement est de 6 jours.

11. Procédé selon la revendication 10, dans lequel les cellules T modifiées artificiellement sont pour utilisation dans le traitement d'un patient cancéreux, dans lequel le patient cancéreux et le sujet donneur sont de préférence la même personne.

12. Procédé selon la revendication 1, dans lequel le système fermé est un système de sac fermé.

13. Procédé selon la revendication 1 ou 12, dans lequel le sac n'est pas bloqué avec HSA pendant l'étape de transduction.

14. Procédé selon la revendication 1, dans lequel la population de cellules comprend des cellules T naïves, dans lequel de préférence environ 35 à 43 %, au moins environ 35 %, ou au moins environ 43 % de la population de cellules T modifiées artificiellement comprennent des cellules T naïves.
